(19) **Europäisches Patentamt**
**European Patent Office**
**Office européen des brevets**

(11) **EP 4 643 867 A1**

(12) **EUROPEAN PATENT APPLICATION**

(43) Date of publication:
**05.11.2025 Bulletin 2025/45**

(21) Application number: **24173541.4**

(22) Date of filing: **30.04.2024**

(51) International Patent Classification (IPC):
**A61K 38/00** (2006.01)   **A61K 38/16** (2006.01)
**C07K 14/00** (2006.01)   **C07K 14/705** (2006.01)
**A61K 38/18** (2006.01)

(52) Cooperative Patent Classification (CPC):
**C07K 14/71; A61K 38/00; A61K 38/16;**
**A61K 38/1866**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Eberhard Karls Universität Tübingen**
**(Medizinische Fakultät)**
**72074 Tübingen (DE)**

(72) Inventors:
 • **Skokowa, Julia**
  **72127 Kusterdingen (DE)**

 • **El Gamacy, Mohammad**
  **72076 Tübingen (DE)**
 • **Maksymenko, Kateryna**
  **72076 Tübingen (DE)**
 • **Pashkovskaia, Natalia**
  **72076 Tübingen (DE)**

(74) Representative: **Witte, Weller & Partner**
**Patentanwälte mbB**
**Postfach 10 54 62**
**70047 Stuttgart (DE)**

Remarks:
The complete document including Reference
Table(s) and the Sequence Listing(s) can be
downloaded from the EPO website

(54) **VEGF-BINDING PROTEIN**

(57) The present invention relates to a polypeptide configured to assemble into an inhibitor of vascular endothelial growth factor (VEGF), a protein comprising said polypeptide, a nucleic acid molecule encoding said polypeptide or protein, an expression vector comprising the nucleic acid molecule, a recombinant host cell comprising said polypeptide, protein, nucleic acid molecule and/or expression vector, a pharmaceutical composition comprising the said polypeptide, protein, nucleic acid molecule, expression vector and/or host cell, and to a kit.

**EP 4 643 867 A1**

**Description**

The present invention relates to a polypeptide configured to assemble into an inhibitor of vascular endothelial growth factor (VEGF), a protein comprising said polypeptide, a nucleic acid molecule encoding said polypeptide or protein, an expression vector comprising the nucleic acid molecule, a recombinant host cell comprising said polypeptide, protein, nucleic acid molecule and/or expression vector, a pharmaceutical composition comprising the said polypeptide, protein, nucleic acid molecule, expression vector and/or host cell, and to a kit.

The present invention in particular relates to new proteinic VEGF inhibitors. Said inhibitors were developed by a novel computational approach using scaffolds of complementary shape as starting compounds.

BACKGROUND OF THE INVENTION

Vascular endothelial growth factor (VEGF), originally known as vascular permeability factor (VPF), is a signal protein produced by many cells, that stimulates the formation of blood vessels. VEGF is a key regulator of normal and pathological angiogenesis. VEGF forms a 2:2 complex with the ectodomain of its receptor (VEGFR), which triggers activation of the intracellular receptor tyrosine kinase domain and the subsequent signaling cascades. The term VEGF describes a family of proteins that have different properties. VEGF-A was discovered first, followed by the factors VEGF-B and PGF (placental growth factor) as well as VEGF-C and VEGF-D (both important for the formation of lymphatic vessels). There are also the related viral homologs (VEGF-E) and the VEGF-F present in snake venom.

VEGF-A and the corresponding receptors are rapidly up-regulated after traumatic injury of the central nervous system (CNS). VEGF-A has been implicated with poor prognosis in breast cancer. VEGF-A is released in rheumatoid arthritis. VEGF-A is also important in diabetic retinopathy (DR). VEGF-A plays a role in the disease pathology of the wet form age-related macular degeneration (AMD), which is the leading cause of blindness for the elderly of the industrialized world. Therefore, VEGF is a validated target for anti-cancer, cardiovascular, neurologic and ophthalmic therapies.

Current anti-VEGF therapies include both small molecules and protein-based inhibitors. Small molecules act by inhibiting the VEGFR intracellular kinase domain, however, they are less specific and often toxic. The first protein-based anti-VEGF drug, a monoclonal antibody named bevacizumab, was approved in 2004. However, only approximately 10-15% of patients benefit from bevacizumab therapy. Ranibizumab is a fragment of bevacizumab. It is used to treat wet macular degeneration, an eye disease that often leads to blindness and is frequently associated with the formation of new blood vessels. Like the other VEGF inhibitors, it is also used in the treatment of cystoid macular oedema as a result of branch retinal vein occlusion (BRVO) or central retinal vein occlusion (CRVO). Furthermore, protein-based inhibitors, which block the VEGF:VEGFR interaction, are disadvantageously large, complex molecules that are post-translationally modified. Their production and conversion into a ready-to-use drug is therefore complex and costly.

It is therefore one of the underlying objectives of the invention to provide new VEGF inhibitors with which the problems or disadvantages of the current active substances can be reduced and possibly even avoided. In particular, such new VEGF inhibitors are to be provided which are easier to manufacture and have a reduced size and can therefore be more easily converted into a drug.

SUMMARY OF THE INVENTION

The object underlying the invention is solved by the provision of a polypeptide configured to assemble into an inhibitor of vascular endothelial growth factor (VEGF), said inhibitor specifically binds to the receptor binding site of VEGF.

The object underlying the invention is solved by the provision of a protein comprising the polypeptide according to the invention.

According to the invention, the term "polypeptide" refers to a series of amino acid residues, connected one to the other typically by peptide bonds between the alpha-amino and carbonyl groups of the adjacent amino acids. The length of the polypeptide is not critical to the invention as long as the correct epitopes are maintained, e.g., the epitope(s) which bind(s) to the receptor binding site of VEGF. The term "polypeptide" is meant to refer to molecules containing more than about 30 amino acid residues. In particular, the polypeptides according to the invention comprise approx. 100 - 200 amino acids, preferably, 120 - 180 amino acids. According to the invention, the term "protein" as used herein, describes a macromolecule comprising one or more polypeptide chains.

According to the invention, an "inhibitor" generally refers to a substance that decreases or prevents the activity of a target molecule. Specifically, it refers to a protein that decreases or prevents the activity of VEGF by blocking its ability to bind or activate its receptor (VEGFR). This inhibition can be achieved through the inhibitor binding directly to a specific part of VEGF that normally interacts with its receptor. By doing so, the inhibitor effectively blocks the binding site, preventing the VEGF from attaching to and activating its receptor VEGFR.

According to the invention, "specifically binding to" is defined as the general interaction between two molecules where one molecule (the binder) recognizes and binds to a distinct and predefined region (epitope) on the other molecule (the target), with a significantly higher affinity and selectivity compared to interactions with other molecules or epitopes. Specifically, the binder is represented by the inhibitor according to the invention, and the target is VEGF. The binding occurs under stringent conditions that mimic physiological conditions or under enhanced conditions that further discriminate specific from non-specific interactions. Stringent conditions may include factors such as temperature, ionic strength, and pH that are optimized to reduce or prevent the binding of molecules that interact weakly or non-specifically. A specific binding can be determined via various measurements or assays, e.g. affinity measurements, competition assays, mutational analyses, etc.

According to the invention, "receptor binding site" refers to a distinct region of the target, in particular VEGF, that is uniquely suited to interact with a specific region of the target's receptor, in particular VEGFR.

According to the invention "assembly" refers to a process by which a polypeptide chain folds and combines, often with other polypeptide chains, to form a functional protein structure. In particular, it refers to a biochemical process where, under suitable conditions, one chain of the polypeptide of the invention folds, and, if applicable, combines with another chain of the polypeptide of the invention, into a distinct and stable three-dimensional structure that constitutes a functional inhibitor protein. This process may include the transition from linear sequences of amino acids (primary structure) to complex structures involving alpha helices and beta sheets (secondary structures), which further fold into unique three-dimensional configurations (tertiary structures). In multimeric proteins, multiple polypeptides (subunits) associate to form a functional complex (quaternary structure). It may also involve non-covalent interactions such as hydrogen bonds, ionic bonds, hydrophobic interactions, and van der Waals forces primarily drive the assembly process. In some cases, covalent disulfide bonds may also stabilize the structure.

According to the invention, the vascular endothelial growth factor (VEGF) includes all members of mammalian VEGF family, such as VEGF-A, placenta growth factor (PGF), VEGF-B, VEGF-C and VEGF-D, and all isoforms thereof, wherein, however, VEGF-A is preferred. In particular, it is referred to human VEGF.

It is self-evident to a person skilled in the art that all features, properties, advantages, etc., disclosed for the polypeptide according to the invention apply correspondingly to the protein according to the invention, without the need for explicit reference thereto.

The problem underlying the invention is hereby completely solved.

The inventors succeeded in designing protein inhibitors blocking the receptor-binding site of the VEGF. Owing to their small size, the new inhibitors have superior pharmacokinetic properties compared to approved anti-VEGF antibodies. The inventors used a new computational strategy which obviates steps of extensive empirical optimization or *in vitro* screening. A "dock-and-design" pipeline retrieved VEGF-complementary scaffolds from a protein structure database to a given query epitope, where the scaffold is mutated to carve a binding site *de novo.* The docking step utilizes a novel fingerprint that greatly simplifies and accelerates the surface complementarity evaluation.

The polypeptides or inhibitors according to the invention bind VEGF with nanomolar affinities and show anti-VEGF activity in primary endothelial cell survival assay and in leukemia cell proliferation assay. Additionally, the new polypeptides or inhibitors inhibit microvasculature formation in a tissue culture model. Finally, the polypeptides or inhibitors are effective in reducing the size of tumor in glioma zebrafish xenograft model.

The inventors aimed to design inhibitors based on simple, single-domain scaffolds. Having tested several candidates, they identified proteins that showed strong VEGF binding, anti-VEGF activity in cell-based and *in vivo* assays, highlighting their therapeutic potential.

VEGF is a key angiogenic molecule involved in the pathogenesis of diverse cancers, cardiovascular, neurologic and ophthalmic diseases. By developing said novel VEGF binders or inhibitors potent and promising new therapeutic agents are provided, superior to existing VEGF inhibitors.

In an embodiment of the invention the polypeptide is a non-immunoglobulin-derived polypeptide.

This measure has the advantage that the problems associated with the current anti-VEGF antibodies, such as bevacizumab and ranibizumab, are avoided. In particular, antibody-based inhibitors blocking VEGF:VEGFR interaction, are large, complex molecules that are post-translationally modified. This is not the case with the polypeptides or inhibitor proteins of the invention which are small, e.g. ranging from approx. 15 to 20 kDa, devoid of any post-translational modifications, readily expressed and purified. "Non-immunoglobulin-derived" means that the polypeptide does not include an antibody, T cell receptor, or a fragments thereof.

In yet another embodiment of the invention the polypeptide comprises the following amino acid sequence SEQ ID NO: 1:

LPTA$X_1$EVQ$X_2$LMARYIEL$X_3$D$X_4$GDIEAIVQMYADDATVEAPFG$X_5$PPI

HGRERIAYFYRRMLG$X_6$GIARATLTGPVRASHNGTGAMPFRVE$X_7$V$X_8$NGQP$X_9$AMD$X_{10}$RVEMRFDEHGRIQTMQAYW$X_{11}X_{12}$VN$X_{13}$SVREP,

wherein

X₁ = Q or E
X₂ = G or K
X₃ = M or L
X₄ = V or K
X₅ = A or S
X₆ = G or E
X₇ = F or Y
X₈ = F or L
X₉ = Y or F or V
X₁₀ = V or I
X₁₁ = S or D
X₁₂ = W or E
X₁₃ = L or I or V or D.

In still another embodiment of the invention the polypeptide comprises the following amino acid sequence SEQ ID NO: 2:

**X₁X₂**STNISPKQGLDKAKYFSGKWYLTHVLIKDP**X₃X₄**V**X₅**QFC**X₆**SFTP
RESDGTVK**X₇**A**X₈**Y**X₉**YLAIKKTS**X₁₀**YAIGEGKLESSGLQYTAT**X₁₁**K**X₁₂**VDKKKAVLKE**X₁₃**DER**X₁₄**SYT**X₁₅**TVLEADDSSALTH**X₁₆X₁₇X₁₈**REGS
KDYGDYY**X₁₉**VLTHQKDAEPSAKVKSAVTQAGLQLSQFVGTKDLGC
QYDDQFTSL

wherein

X₁ = D or missing

X₂ = C or missing

X₃ = V or K

X₄ = A or I

X₅ = T or S

X₆ = S or A

X₇ = E or I

X₈ = I or V

X₉ = V or L

X₁₀ = E or D

X₁₁ = F or S

X₁₂ = T or V

X₁₃ = W or L

X₁₄ = Y or H

$X_{15}$ = I or V

$X_{16}$ = V or I

$X_{17}$ = C orT

$X_{18}$ = T or V

$X_{19}$ = M or L.

The inventors were able to identify two consensus amino acid sequences derived from the individually developed polypeptides or VEGF inhibitors, respectively, that yields polypeptides that have the desired properties. This measure has the advantage that a person skilled in the art has a range of variation options. That is, at positions $X_1$-$X_{13}$ (SEQ ID NO: 1) or $X_1$-$X_{19}$ (SEQ ID NO: 2), the respective amino acids indicated can be used as desired. It is understood that according to the invention, all arbitrary and conceivable combinations of $X_1$-$X_{13}$ (SEQ ID NO: 1) or $X_1$-$X_{19}$ (SEQ ID NO: 2) as indicated are encompassed and disclosed.

The inventors have used two different scaffolds to develop the polypeptides according to the invention, named 'Sam' and 'Sima'. The use of 'Sam' resulted in consensus sequence SEQ ID NO: 1 (the 'Sam consensus sequence'), and the use of 'Sima' resulted in consensus sequence SEQ ID NO: 2 (the 'Sima consensus sequence').

Of particular advantage is that the polypeptides according to the invention are relatively short and small, which facilitates their production and assembly into a protein. The 'Sam' polypeptides have a length of about 100 - about 150 amino acids, preferably of about 110 - about 130 amino acids, most preferably of about 128 amino acids. The 'Sima' amino acids have a length of about 150 - about 200 amino acids, preferably of about 170 - 180 amino acids, most preferably of about 177 and/or 179 amino acids.

In another embodiment the polypeptide according to the invention comprises the following amino acid sequence SEQ ID NO: 3:

LPTAEEVQKLMARYIELMDVGDIEAIVQMYADDATVEAPFGAPPIHGRERIAYFYR
RMLGGGIARATLTGPVRASHNGTGAMPFRVEYVFNGQPFAMDVRVEMRFDEH
GRIQTMQAYWDWVNLSVREP (Sam0.7)

or the following amino acid sequence SEQ ID NO: 4:

STNISPKQGLDKAKYFSGKWYLTHVLIKDPKIVSQFCASFTPRESDGTVKIAVYLY
LAIKKTSDYAIGEGKLESSGLQYTATSKVVDKKKAVLKELDERHSYTVTVLEADDS
SALTHITVREGSKDYGDYYLVLTHQKDAEPSAKVKSAVTQAGLQLSQFVGTKDL
GCQYDDQFTSL (Sima3.2)

or the following amino acid sequence SEQ ID NO: 5:

LPTAQEVQGLMARYIELMDVGDIEAIVQMYADDATVEAPFGAPPIHGRERIAYFY
RRMLGGGIARATLTGPVRASHNGTGAMPFRVEFVFNGQPYAMDVRVEMRFDEH
GRIQTMQAYWSWVNLSVREP (Sam0.1)

or the following amino acid sequence SEQ ID NO: 6:

LPTAEEVQKLMARYIELMDVGDIEAIVQMYADDATVEAPFGAPPIHGRERIAYFYR
RMLGEGIARATLTGPVRASHNGTGAMPFRVEYVFNGQPYAMDVRVEMRFDEHG
RIQTMQAYWSEVNDSVREP (Sam0.2)

or the following amino acid sequence SEQ ID NO: 7:

LPTAEEVQKLMARYIELMDKGDIEAIVQMYADDATVEAPFGAPPIHGRERIAYFYR
RMLGEGIARATLTGPVRASHNGTGAMPFRVEFVFNGQPYAMDVRVEMRFDEHG
RIQTMQAYWSWVNLSVREP (Sam0.3)

or the following amino acid sequence SEQ ID NO: 8:

LPTAEEVQKLMARYIELLDVGDIEAIVQMYADDATVEAPFGSPPIHGRERIAYFYR
RMLGEGIARATLTGPVRASHNGTGAMPFRVEYVLNGQPVAMDIRVEMRFDEHG
RIQTMQAYWSWVNVSVREP (Sam0.4)

or the following amino acid sequence SEQ ID NO: 9:

LPTAEEVQKLMARYIELMDVGDIEAIVQMYADDATVEAPFGAPPIHGRERIAYFYR
RMLGGGIARATLTGPVRASHNGTGAMPFRVEYVFNGQPYAMDVRVEMRFDEH
GRIQTMQAYWSWVNLSVREP (Sam0.5)

or the following amino acid sequence SEQ ID NO: 10:

LPTAEEVQKLMARYIELMDVGDIEAIVQMYADDATVEAPFGAPPIHGRERIAYFYR
RMLGGGIARATLTGPVRASHNGTGAMPFRVEFVFNGQPFAMDVRVEMRFDEHG
RIQTMQAYWSWVNLSVREP (Sam0.6)

or the following amino acid sequence SEQ ID NO: 11:

LPTAEEVQKLMARYIELMDVGDIEAIVQMYADDATVEAPFGAPPIHGRERIAYFYR
RMLGGGIARATLTGPVRASHNGTGAMPFRVEYVFNGQPYAMDVRVEMRFDEH
GRIQTMQAYWSWVNISVREP (Sam0.8)

or the following amino acid sequence SEQ ID NO: 12:

DCSTNISPKQGLDKAKYFSGKWYLTHVLIKDPVAVTQFCSSFTPRESDGTVKEAI
YVYLAIKKTSEYAIGEGKLESSGLQYTATFKTVDKKKAVLKEWDERYSYTITVLEA
DDSSALTHVCTREGSKDYGDYYHVLTHQKDAEPSAKVKSAVTQAGLQLSQFVG
TKDLGCQYDDQFTSL (Sima1.1)

or the following amino acid sequence SEQ ID NO: 13:

DCSTNISPKQGLDKAKYFSGKWYLTHVLIKDPVAVTQFCSSFTPRESDGTVKEAI
YVYLAIKKTSEYAIGEGKLESSGLQYTATFKTVDKKKAVLKEWDERYSYTITVLEA
DDSSALTHVCTREGSKDYGDYYMVLTHQKDAEPSAKVKSAVTQAGLQLSQFVG
TKDLGCQYDDQFTSL (Sima2.1)

or the following amino acid sequence SEQ ID NO: 14:

DCSTNISPKQGLDKAKYFSGKWYLTHVLIKDPKIVSQFCASFTPRESDGTVKIAVY
LYLAIKKTSDYAIGEGKLESSGLQYTATSKVVDKKKAVLKELDERHSYTVTVLEAD
DSSALTHICVREGSKDYGDYYLVLTHQKDAEPSAKVKSAVTQAGLQLSQFVGTK
DLGCQYDDQFTSL (Sima3.1).

or the following amino acid sequence SEQ ID NO: 15:

DCSTNISPKQGLDKAKYFSGKWYLTHVLIKDPVAVTQFCSSFTPRESDGTVKVAI
YVYLAIKKTSEYAIGEGKLESSGLQYTATFKTVDKKKAVLKEFDERHSYTITVLEAD
DSSALVHVCVREGSKDYGDYYNVLTHQKDAEPSAKVKSAVTQAGLQLSQFVGT
KDLGCQYDDQFTSL (Sima4.1)

or the following amino acid sequence SEQ ID NO: 16:

STNISPKQGLDKAKYFSGKWYLTHVLIKDPVAVTQFCSSFTPRESDGTVKEAIYV
YLAIKKTSEYAIGEGKLESSGLQYTATFKTVDKKKAVLKEWDERYSYTITVLEADD
SSALTHVTTREGSKDYGDYYHVLTHQKDAEPSAKVKSAVTQAGLQLSQFVGTKD
LGCQYDDQFTSL (Sima1.2)

or the following amino acid sequence SEQ ID NO: 17:

STNISPKQGLDKAKYFSGKWYLTHVLIKDPVAVTQFCSSFTPRESDGTVKEAIYV
YLAIKKTSEYAIGEGKLESSGLQYTATFKTVDKKKAVLKEWDERYSYTITVLEADD

SSALTHVTTREGSKDYGDYYMVLTHQKDAEPSAKVKSAVTQAGLQLSQFVGTK
DLGCQYDDQFTSL (Sima2.2)

or the following amino acid sequence SEQ ID NO: 18:

STNISPKQGLDKAKYFSGKWYLTHVLIKDPVAVTQFCSSFTPRESDGTVKVAIYV
YLAIKKTSEYAIGEGKLESSGLQYTATFKTVDKKKAVLKEFDERHSYTITVLEADD
SSALVHVTVREGSKDYGDYYNVLTHQKDAEPSAKVKSAVTQAGLQLSQFVGTK
DLGCQYDDQFTSL (Sima4.2).

This measure provides specific VEGF inhibitors or polypeptides, designated by the inventors as 'Sam0.1', 'Sam0.2', 'Sam0.3', 'Sam0.4', 'Sam0.5', 'Sam0.6', 'Sam0.7', 'Sam0.8', 'Sima1.1', 'Sima2.1', 'Sima3.1', 'Sima4.1', 'Sima1.2', 'Sima2.2', 'Sima3.2', and 'Sima4.2', which are particularly suitable according to their findings in accordance with the invention.

In a particularly preferred embodiment of the invention the polypeptide consists or consists essentially of an amino acid sequence according to SEQ ID NO: 1 to SEQ ID NO: 18.

"Consisting essentially of" shall mean that a polypeptide according to the present invention, in addition to the sequence according to any of SEQ ID NO: 1 to SEQ ID NO:18 contains additional N- and/or C-terminally located stretches of amino acids that are not necessarily forming part of the polypeptide that functions as a VEGF binding epitope.

The polypeptides and/or proteins and/or nucleic acid molecules disclosed in accordance with the present invention may also be in "purified" form. The term "purified" does not require absolute purity; rather, it is intended as a relative definition, and can include preparations that are highly purified or preparations that are only partially purified, as those terms are understood by those of skill in the relevant art.

The polypeptides, proteins and nucleic acids according to the invention may be in "enriched form". As used herein, the term "enriched" means that the concentration of the material is at least about 2, 5, 10, 100, or 1000 times its natural concentration (for example), advantageously 0.01%, by weight, preferably at least about 0.1% by weight. Enriched preparations of about 0.5%, 1%, 5%, 10%, and 20% by weight are also contemplated. The sequences, constructs, vectors, clones, and other materials comprising the present invention can advantageously be in enriched or isolated form.

In an embodiment of the invention the protein comprises one polypeptide having any of the following amino acid sequences: SEQ ID NO: 3 (Sam0.7), SEQ ID NO: 5 (Sam0.1), SEQ ID NO: 6 (Sam0.2), SEQ ID NO: 7 (Sam0.3), SEQ ID NO: 8 (Sam0.4), SEQ ID NO: 9 (Sam0.5), SEQ ID NO: 10 (Sam0.6), SEQ ID NO: 11 (Sam0.8).

This embodiment takes into account the inventors' realization that all 'Sam'-derived proteins or polypeptides are monomeric.

In an other embodiment of the invention the protein comprises one, *two* or *four* polypeptide(s) having any of the following amino acid sequences: SEQ ID NO: 4 (Sima3.2), SEQ ID NO: 12 (Sima1.1), SEQ ID NO: 13 (Sima2.1), SEQ ID NO: 14 (Sima3.1) SEQ ID NO: 15 (Sima4.1), SEQ ID NO: 16 (Sima1.2), SEQ ID NO: 17 (Sima2.2), SEQ ID NO: 18 (Sima4.2).

This embodiment, in turn, takes into account the inventors' realization that all 'Sima'-derived proteins or polypeptides form monomeric, dimeric, or tetrameric forms.

Another subject-matter of the invention relates to the polypeptide and/or the protein according to the invention for use as a medicament, preferably in the treatment of a disease, further preferably selected from the group consisting of: cancer, cardiovascular disease, neurologic disease and ophthalmic disease.

Another subject-matter of the invention relates to a nucleic acid molecule encoding for the polypeptide and/or protein according to the invention, optionally linked to a promoter sequence.

As used herein the term "nucleic acid molecule" or, synonymously "polynucleotide" coding for (or encoding) a polypeptide/protein refers to a nucleotide sequence coding for the protein/polypeptide including artificial (man-made) start and stop codons compatible for the biological system the sequence is to be expressed. The term "promoter" means a region of DNA involved in the binding of the RNA polymerase to initiate transcription.

The polynucleotide or nucleic acid molecule coding for said polypeptide or protein may be synthetically constructed or may be naturally occurring. The nucleic acid or polynucleotide may be, for example, DNA, cDNA, PNA, RNA or combinations thereof, either single- and/or double- stranded, or native or stabilized forms of polynucleotides, such as, for example, polynucleotides with a phosphorothioate backbone, and it may or may not contain introns so long as it codes for the polypeptide. Of course, only peptides that contain naturally occurring amino acid residues joined by naturally occurring peptide bonds are encodable by a polynucleotide.

A still further aspect of the invention provides a vector, such as an expression vector, capable of expressing the polypeptide and/or protein according to the invention.

The features, characteristics, advantages, and embodiments disclosed for the polypeptide and protein according to the invention apply to the nucleic acid molecule and (expression) vector correspondingly.

Another subject-matter of the invention relates to a recombinant host cell comprising the polypeptide or protein according to the invention, or the nucleic acid or the expression vector according to the invention, wherein said host cell preferably is selected from a bacterial (e.g., *E. coli),* yeast, insect, mammalian, or human cell.

The features, characteristics, advantages and embodiments disclosed for the polypeptide and protein according to the invention apply to the host cell correspondingly.

Another subject-matter according to the invention relates to a pharmaceutical composition comprising at least one active ingredient selected from the group consisting of the polypeptide according to the invention, the protein according to the invention, the nucleic acid or the expression vector according to the invention, and the recombinant host cell according to the invention, optionally a pharmaceutically acceptable carrier, and further optionally, pharmaceutically acceptable excipients and/or stabilizers. The pharmaceutical composition is preferably designated for the treatment of a disease, further preferably selected from the group consisting of: cancer, cardiovascular disease, neurologic disease, and ophthalmic disease.

A still further subject-matter according to the invention relates to a kit comprising:

(a) a container comprising the polypeptide, or the protein, or the nucleic acid molecule, or the expression vector, or the recombinant host cell, or the pharmaceutical composition according to the invention, in solution or in lyophilized formulation;

(b) optionally, a second container containing a diluent or reconstituting solution for the lyophilized formulation;

(c) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The pharmaceutical composition is preferably lyophilized.

The kit of the present invention preferably comprises a lyophilized formulation of the present invention in a suitable container and instructions for its reconstitution and/or use. Suitable containers include, for example, bottles, vials (e.g., dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

A still further subject-matter of the invention is the use of the polypeptide according to the invention for reconstituting a VEGF-binding and/or VEGF-inhibiting protein, preferably a monomeric, dimeric or tetrameric protein.

The features, characteristics, advantages and embodiments disclosed for the polypeptide or protein recited at the outset according to the invention apply to said use correspondingly.

A yet further subject matter of the invention relates to a method for identifying binders to a target structure, comprising the following steps:

1. Providing a target structure comprising an epitope configured for a specific binding to a receptor-like molecule,

2. Identifying a protein structure with a surface patch that is highly complementary to the epitope on the target molecule to obtain a complex of protein structure and target molecule;

3. Subjecting the complex to a single-sided interface design where mutations are introduced to the protein structure to create tight molecular interfaces at the complementary interface, and

4. Isolating binders of the target structure from the obtained mutated protein structure and, optionally, identifying the amino acid sequences thereof.

The VEGF inhibitors were developed by afore-mentioned novel computational approach based on scaffolds of complementary shape as starting elements. Said approach, however, can be generalized to any binders and target structures, as reflected in above-identified method according to the invention.

Starting by the structure of a target molecule (method step 1), the overall pipeline for identifying binders against a specific epitope on the target molecule is composed of three following distinct steps:

*1. Steric docking* (method step 2)
The aim of this step is to identify existing protein structures with (subject) surface patches that are highly complementary to the selected (query) epitope on the target molecule (such as VEGF). This docking is done using simplified descriptors of each surface patch. These descriptors are constructed in the form of 2D matrices that describe a cylindrical cut of the molecular surface. These matrices have two key properties: i) they can describe surface patches on either a query or target structures without being sensitive to the particular patch's 3D position or 3D orientation, ii) if two surface patches are ideally complementary, the dissimilarity between their matrices will high. However, if one of the two matrices is axially inverted, it will perfectly overlap with its non-inverted counterpart. Given these two properties, to find structures with a putatively complementary (subject) surface patch against a target (query) epitope, the following steps are performed:

a. A dot-surface representation is derived for all subject structures from a protein structure database (e.g. the Protein Data Bank). This creates a database of high-resolution molecular surfaces to search across.

b. The 2D fingerprints are generated for overlapping surface patches of each structure, resulting in 2D matrices describing tens of thousands of surface patches for each structure in the database.

c. The inverse 2D fingerprint is generated for a desired epitope of the molecular surface on the target molecule.

d. A large-scale comparison between the inverted query fingerprint and all the subject fingerprints, whereby, the subject fingerprints with the highest similarity to the inverted query finger represent the part of the subject structure with high complementarity to the target epitope.

e. This subject structure is then docked to the target structure at the identified highly complementary site.

*2. Interface design* (method step 3)

This docked complex then undergoes single-sided interface design, where mutations are only introduced to the subject structure to create tight molecular interfaces at the complementary interface. This step results in thousands of design models with putative binding capacity.

*3. Molecular dynamics-based filtering* (method step 4)

In order to filter out only a few candidates to experimental evaluate for binding, molecular dynamics simulations are used an orthogonal filter to judge the stability of the designed binding interfaces. Specifically, two schemes of non-equilibrium molecular dynamics are used that either various temperature schemes or steering forces are applied to accelerate the evaluation of the intermolecular interactions' strength. At the end of this step, designs leading to the most stable complexes *in silico* are selected for *in vitro* validation.

The features, characteristics, advantages and embodiments disclosed for the polypeptide or protein recited at the outset according to the invention apply to said use correspondingly.

The invention is now further described and explained in further detail by referring to the following non-limiting examples and figures:

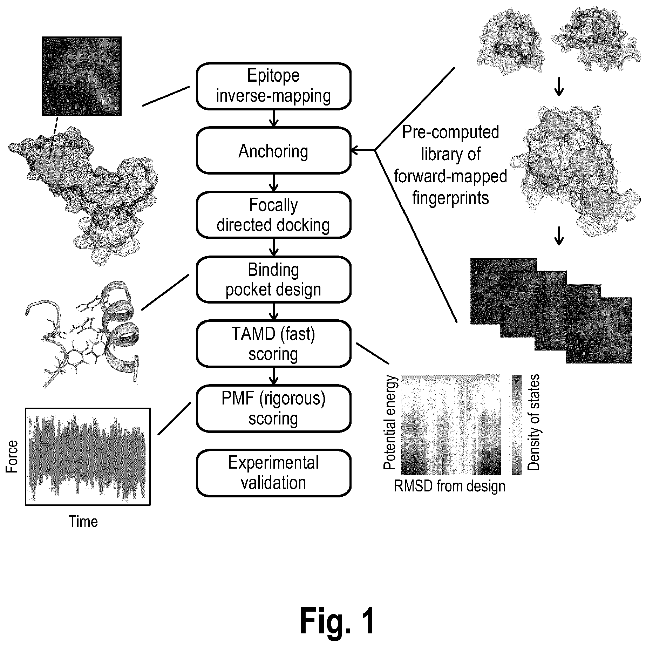

Fig. 1:     Design strategy of epitope-directed binders. The design workflow primarily relies on simplifying the complexity of the docking process and decoupling it from the design stage. The docking step particularly is based on a novel surface mesh fingerprinting protocol that analytically defines a complementary surface fingerprint and trivially matches it to a query surface. This is followed by steps of high-resolution docking, sequence design, and molecular dynamics-based testing of binding interface stability. TAMD - temperature-accelerated molecular dynamics. PMF - potential of mean force. RMSD - root-mean-square deviation.

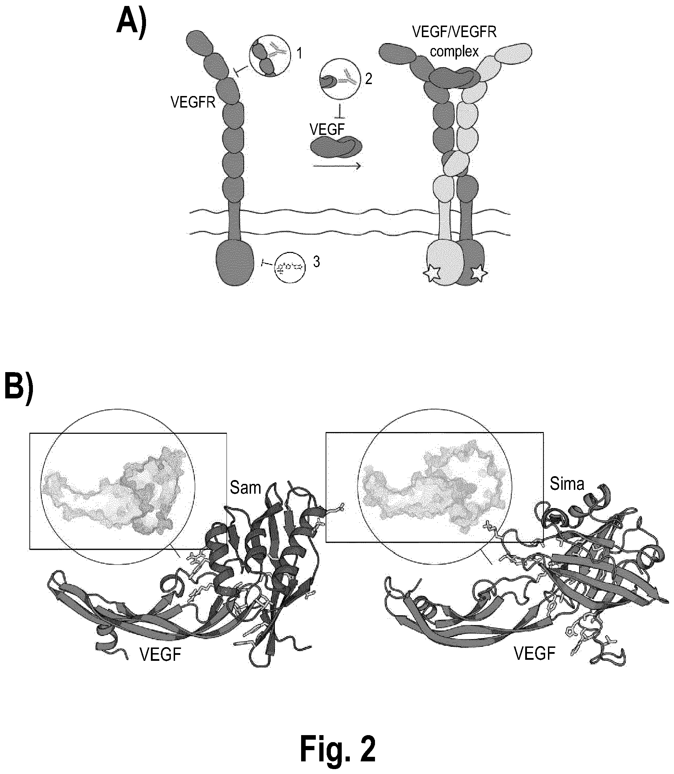

Fig. 2:     Mechanism of VEGFR activation and strategies for its inhibition. (A) VEGFR subunit is composed of 7 extracellular domains, a transmembrane segment, and an intracellular kinase domain. VEGFR is activated when dimeric VEGF binds, and in turn, dimerizes two receptor subunits. This dimeric receptor configuration triggers the intracellular domain kinase activity. VEGFR signaling can be inhibited via: 1) protein-based quenching of the ligand binding site on the receptor surface, 2) protein-based sequestration of VEGF itself at its receptor binding site, or 3) inhibition of the kinase activity through the use of small molecules. (B) Anti-VEGF binders were designed based on two different scaffolds, Sam and Sima, that showed a high shape complementarity to the receptor-binding site of VEGF.

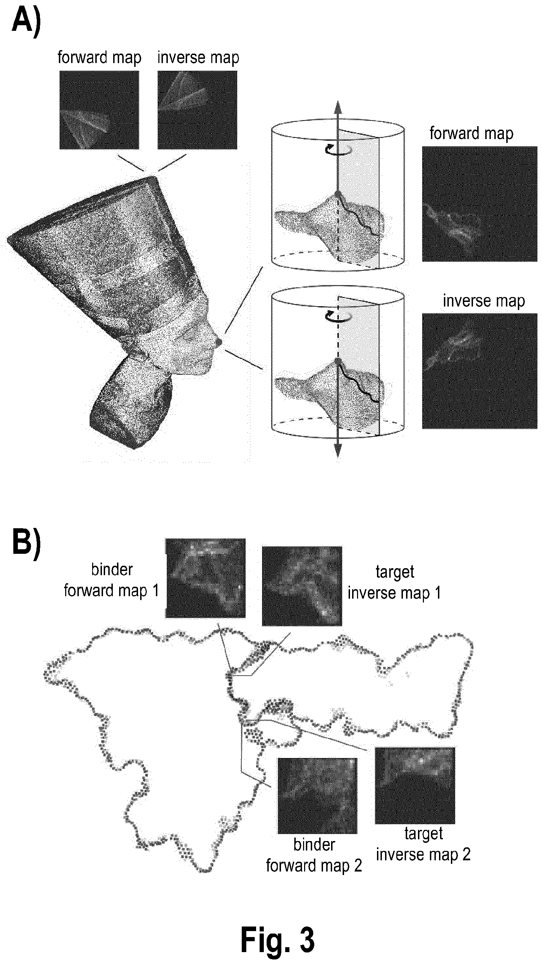

Fig. 3:     The HECTOR fingerprint captures local surface complementarity. (A) The HECTOR mapper takes a dot-surface of an object (e.g. molecular surface of a protein) as input. Surface patches are extracted around a selected dot (e.g. dot at the center of the face and crown patches from Nefertiti Bust). A map is compiled by rotational accumulation of dot density on a matrix. Depending if the surface normal pointing outwards or inwards of an object, the map is defined as "forward" or "inverse". (B) Forward and inverse maps at highly complementary interfaces are highly similar. Shown is an example of a tight interface between VEGF and its antibody (bevacizumab).

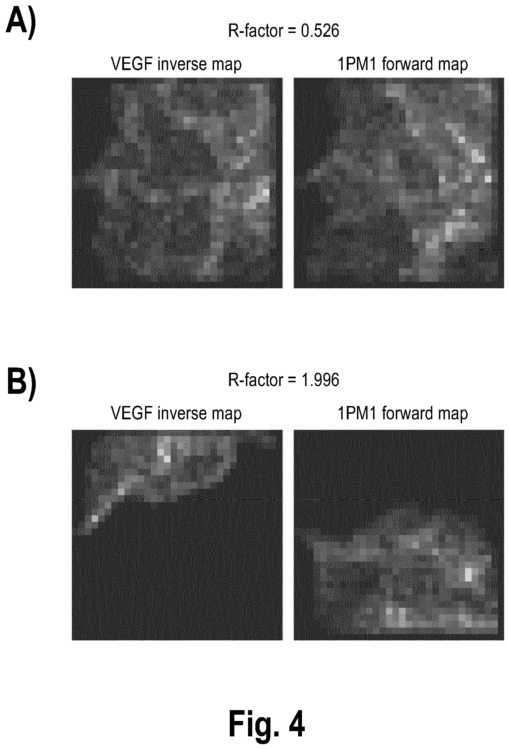

Fig. 4:     Representative fingerprints for surface patches with low and high R-factor. Inverse fingerprints (maps) for the surface patches at the receptor-binding site of VEGF were compared with the forward fingerprints for surface patches of a potential scaffold (PDB: 1 PM1). (A) An example of two fingerprints corresponding to two surface patches with high shape complementarity (low R-factor). (B) An example of two fingerprints corresponding to two surface patches with low shape complementarity (high R-factor).

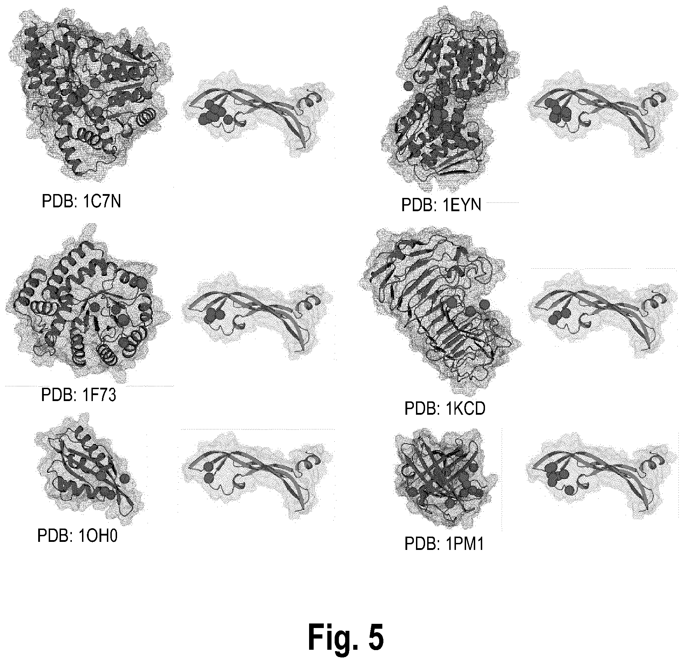

Fig. 5:    PDB hits with high shape complementarity to the receptor-binding site of VEGF. The inverse fingerprints corresponding to the surface patches within a binding site of VEGF (patches with y-coordinate >-5) were compared with the fingerprints of six HECTOR hits. Patches with R-factor lower than 0.55 are shown as spheres. Coordinates of a sphere center correspond to coordinates of a patch center.

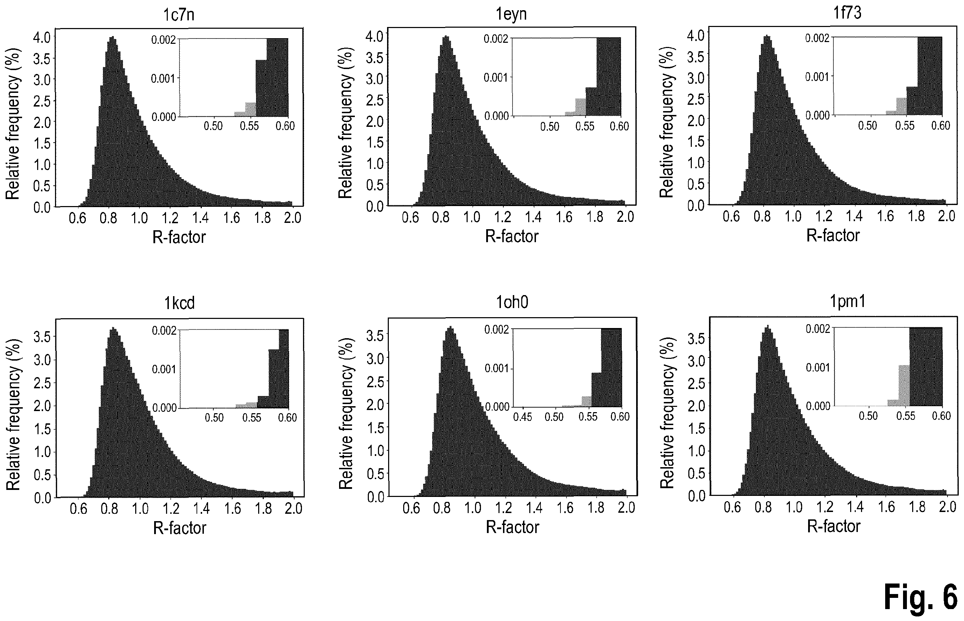

Fig. 6:    R-factor distribution for surface patches of PDB hits with high shape complementarity to the receptor-binding site of VEGF. Data are presented as histograms of R-factor values derived from comparison between the fingerprints of six HECTOR hits and the inverse fingerprints corresponding to the surface patches within a binding site of VEGF. Data points with R-factor values lower than 0.55 are light-colored.

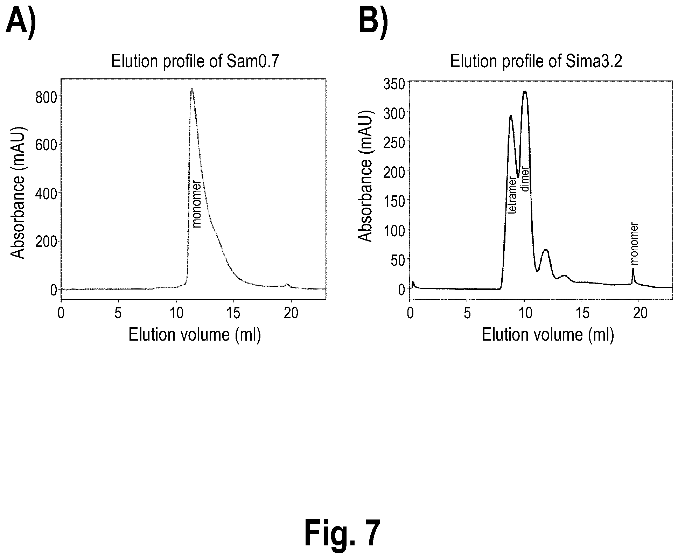

Fig. 7:    Size-exclusion elution profile of the designs. (A) The chromatogram shows Sam0.7 to elute as a monomeric protein. (B) Elution profile of Sima3.2 shows monomeric, dimeric, and tetrameric species. For experimental characterization, dimeric fraction of Sima3.2 was used.

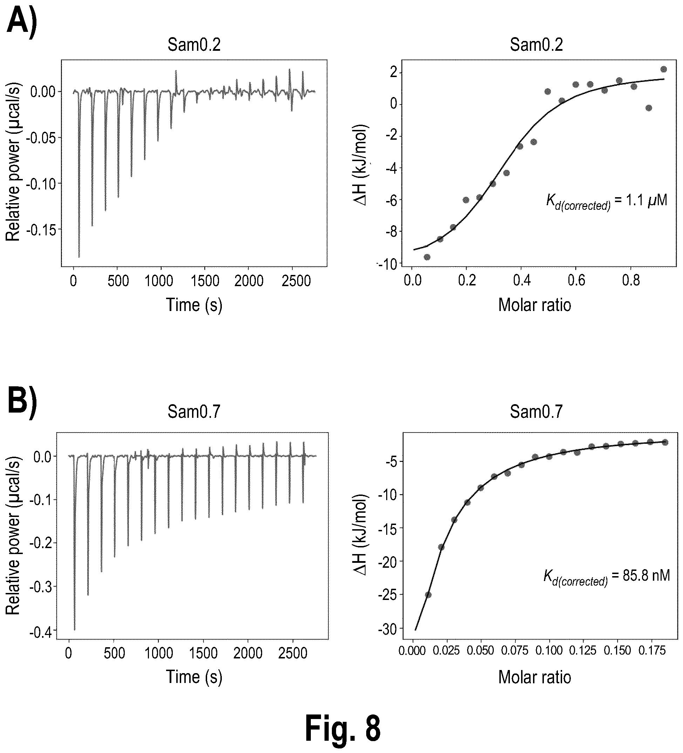

Fig. 8:    Calorigrams (left) and corresponding fitted curves (right) obtained from isothermal titration of VEGF with Sam0.2 (A) and Sam0.7 (B).

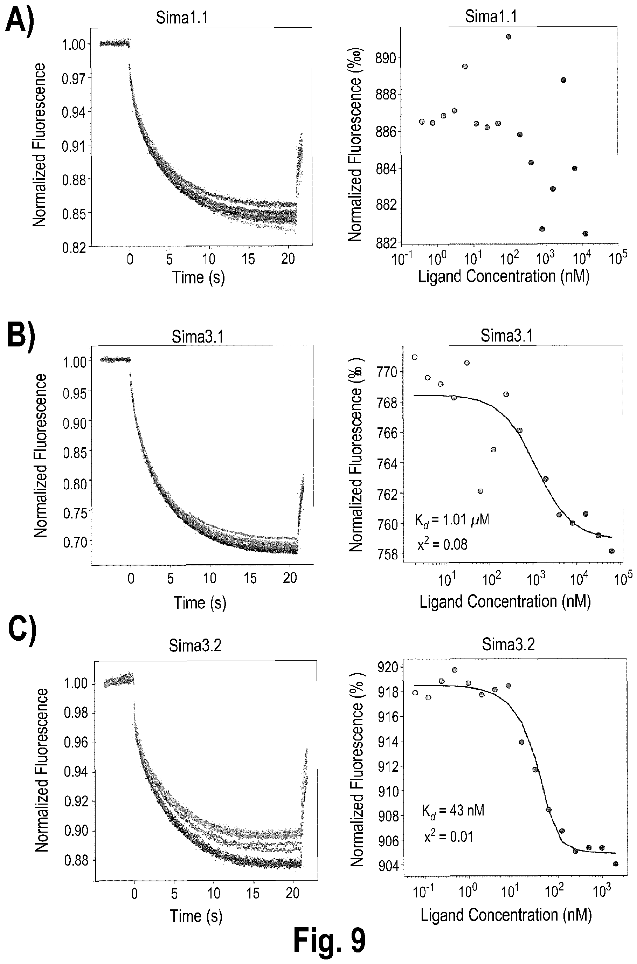

Fig. 9:    Microscale thermophoresis traces (left) and dose-response curves (right) for the binding interaction between VEGF and Sima designs.

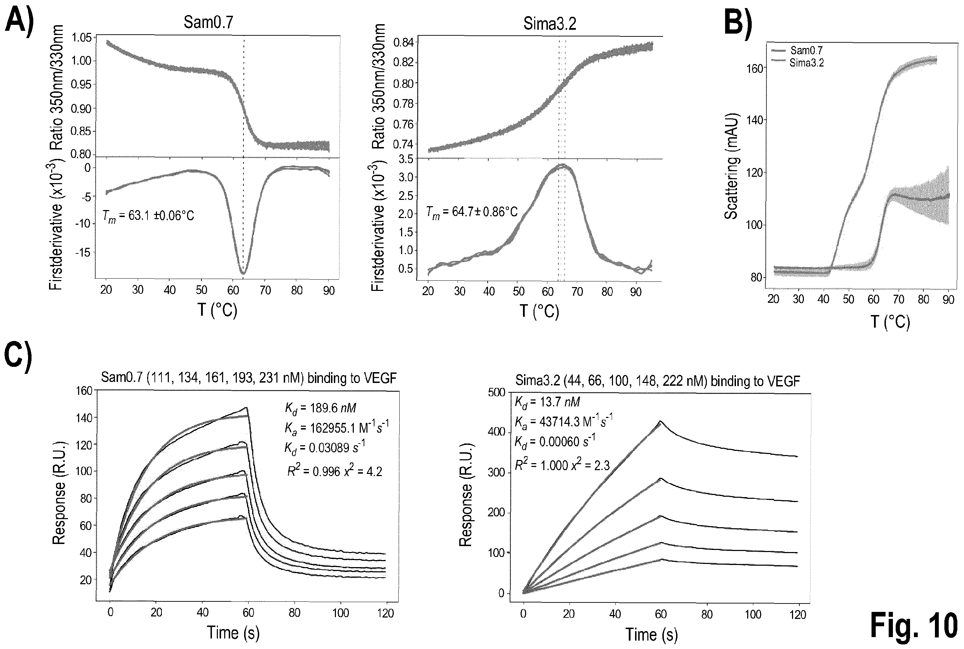

Fig. 10:   Biophysical characterization of the designed proteins. (A) Thermal unfolding curves show melting temperatures (Tm) of both Sam0.7 and Sima3.2 designs to be higher than 60 °C. (B) Light scattering thermograms indicate that Sima3.2 has higher propensity for aggregation with an onset temperature of around 40 °C, compared to an onset temperature of 60 °C for Sam0.7. Shades represent the standard deviation across three replicates. (C) The designed proteins bind VEGF with nanomolar affinity as measured by SPR.

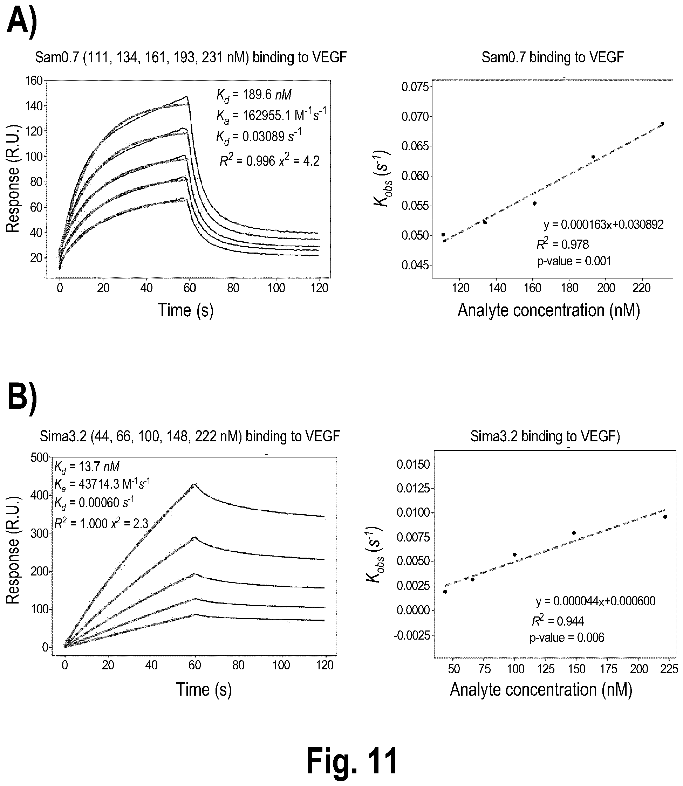

Fig. 11:   SPR sensorgrams of (A) Sam0.7 and (B) Sima3.2 and their binding kinetics fits. Sensograms (shown also in Fig. 10C) and association phase fits are shown (left-side panes; ) against their respective kobs fits (right-side panes).

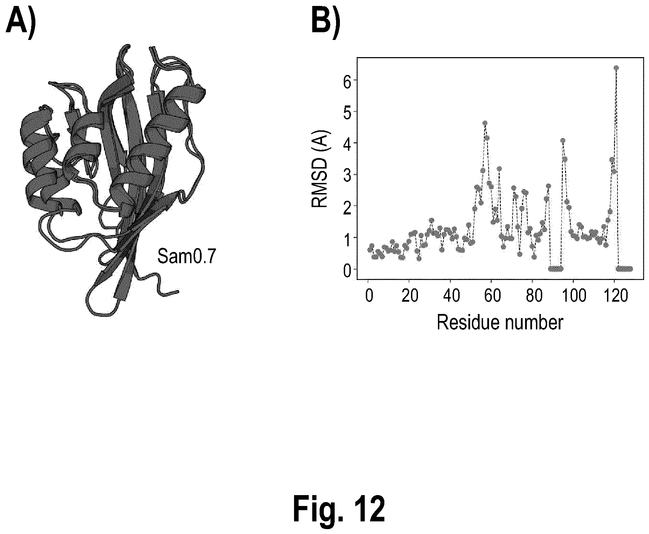

Fig. 12:   Crystal structure of Sam0.7 matches the computational design model with atomic-level accuracy. (A) Superimposition of the Sam0.7 design model and the experimentally determined crystal structure. (B) RMSD between coordinates of the C$\alpha$ atoms in the Sam0.7 design model and corresponding C$\alpha$ atoms in the crystal structure. Residues in gaps are assigned an RMSD value of 0.

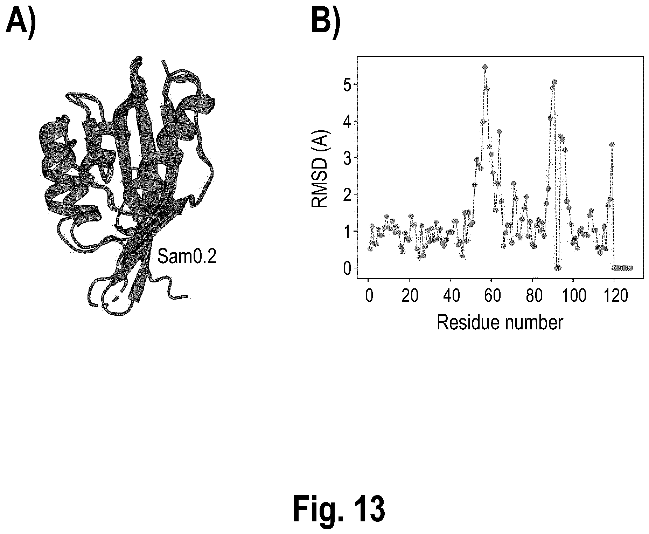

Fig. 13:   Crystal structure of Sam0.2 matches the computational design model with atomic-level accuracy. (A) Superimposition of the Sam0.2 design model and the experimentally determined crystal structure. (B) RMSD between coordinates of the C$\alpha$ atoms in the Sam0.2 design model and corresponding C$\alpha$ atoms in the crystal structure.. Residues in gaps are assigned an RMSD value of 0.

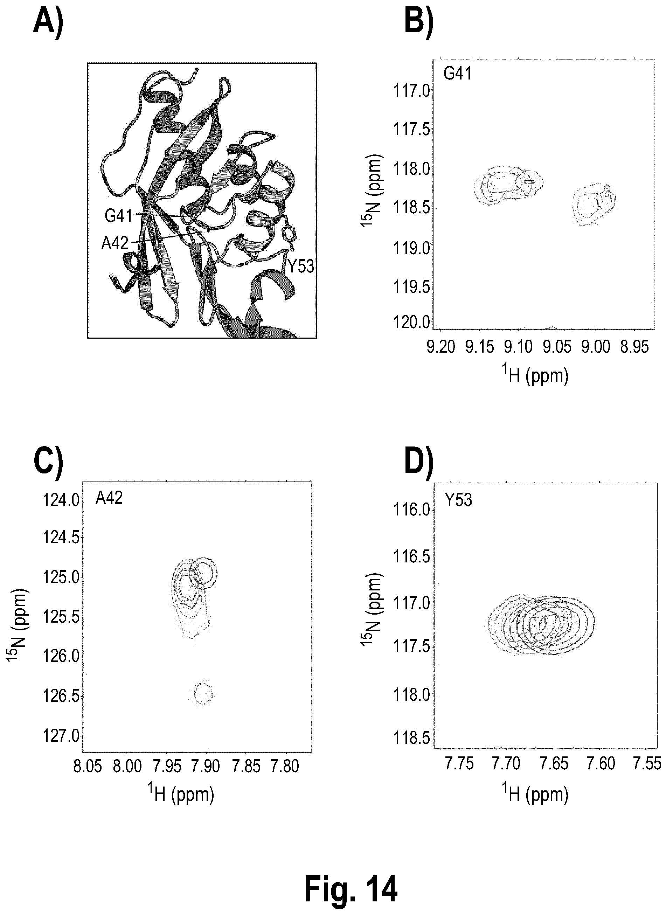

Fig. 14:   VEGF titration causes NMR chemical shift perturbations of Sam0.2. (A) Sequential assignment of 15N-HSQC peaks was achieved for majority of Sam0.2 residues. Although many residues at the outer rim of the binding site could not be assigned, peaks for some residues showed significant chemical shift changes upon VEGF titration. (B-D). The overlaid 2D 1H-15N HSQC spectra for 15N,13C-labelled Sam0.2, residues G41, A42, Y53, in the absence or presence of VEGF at a molar ratio of 1:2 and 1:3.

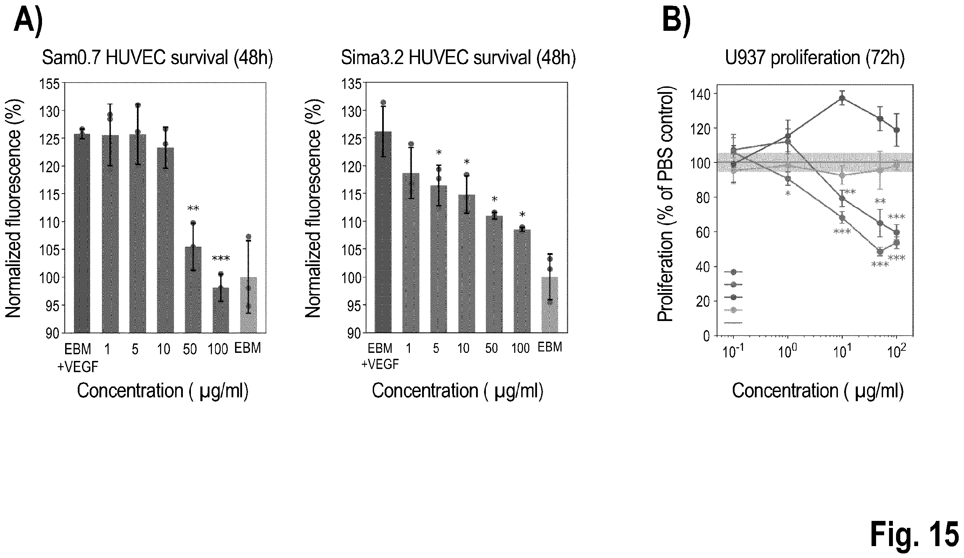

Fig. 15:   The designs inhibit proliferation and survival of VEGF-dependent cells. (A) VEGF-dependent survival of HUVEC primary cells was significantly reduced in a dose-dependent manner by the designed binders. Bars indicate either survival of the cells in an endothelial cell growth basal medium without VEGFor survival of the cells in a basal medium with 30 nM of VEGF, or survival in a basal medium with 30 nM of VEGF and increasing concentrations of Sam0.7 and Sima3.2, respectively. (B) Treatment of U937 acute myeloid leukemia cell line with Sam0.7 and Sima3.2 proteins at low micromolar concentrations decreased the cell growth. In contrast, unmutated scaffolds, Sam_cntrl and Sima_cntrl, did not show any inhibitory activity. Error bars represent the standard deviations across three replicates from one experiment. Statistical significance was calculated using Fisher's one-sided t-test (*, $p \leq 0.05$, **, $p \leq 0.01$, ***, $p \leq 0.001$ vs. the PBS group).

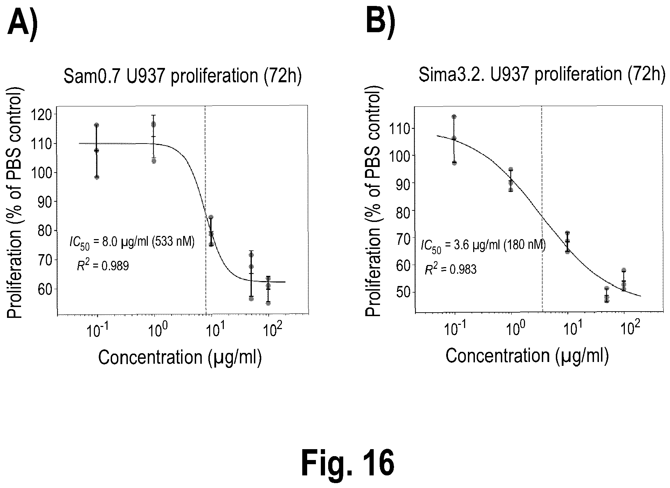

Fig. 16: Proliferation assays using the VEGF-dependent U937 acute myeloid leukemia cell line. Sam0.7 (A) and Sima3.2 (B) suppressed proliferation of U937 cells with IC50 values of 533 nM and 180 nM, respectively. Error bars represent the standard deviation across three replicates.

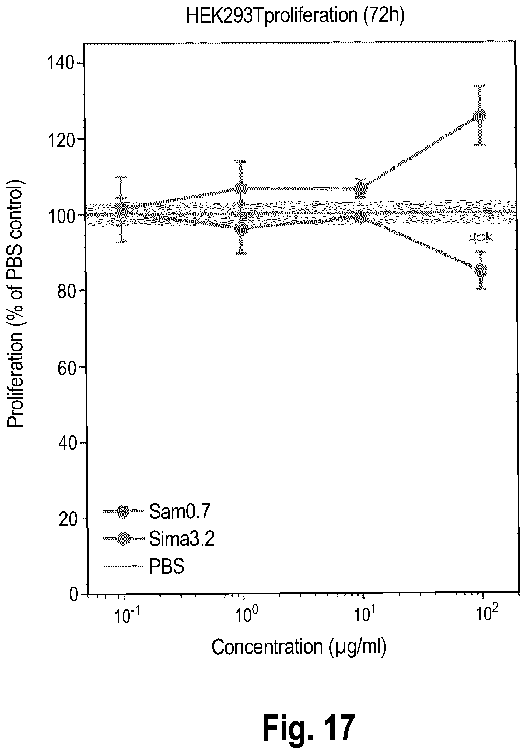

Fig. 17: The designs did not show a strong inhibitory effect on proliferation of the human embryonic kidney cell line. Proliferation of HEK293T cells was mostly not affected by the designed binders. Only treatment with the highest concentration of Sam0.7 (100 $\mu$g/ml) could decrease the cell growth. Error bars represent the standard deviations across three replicates from one experiment. Statistical significance was calculated using Fisher's one-sided t-test (**, $p \leq 0.01$ vs. the PBS group).

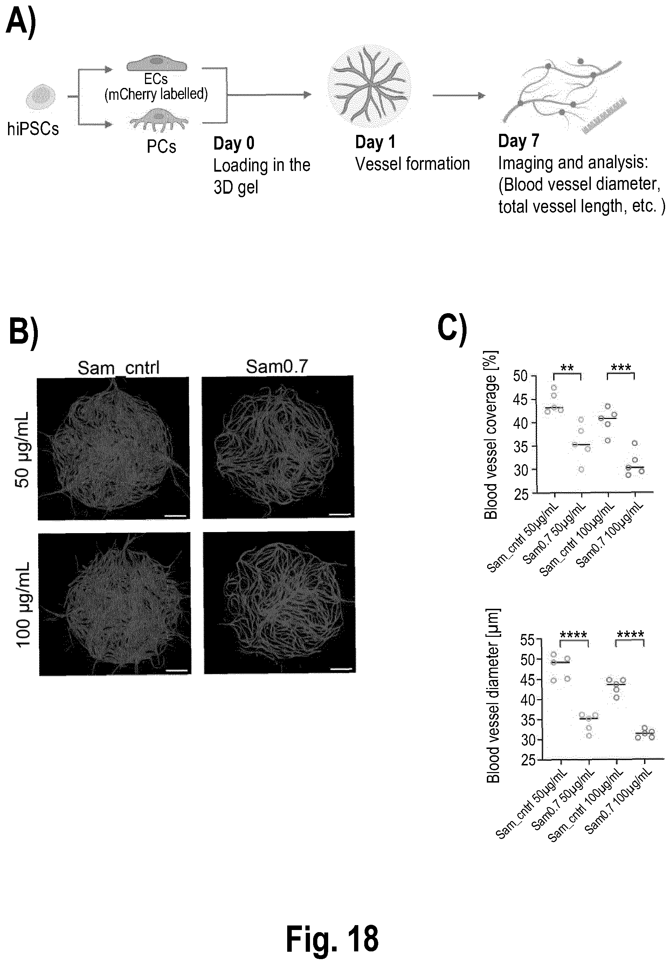

Fig. 18: The designed binder Sam0.7 reduces angiogenesis in vitro. (A) Schematic representation of in vitro microvasculature formation and analysis: human induced pluripotent stem cell (hiPSC)-derived endothelial cells (ECs) and pericytes (PCs) were co-cultured in the fibrin gel with Sam0.7 or Sam_cntrl as a negative control. The formed microvasculature was imaged at day 7, and images were analyzed to calculate microvasculature parameters. The figure was created with BioRender. (B) Representative images showing in vitro microvasculature formation in the presence of Sam0.7 or Sam_cntrl at two working concentrations (50 and 100 $\mu$g/mL). The scale bar is 500 $\mu$m. (C) Quantitative analysis of microvasculature formation. The upper plot shows the percentage of the area covered with blood vessels, and the bottom plot shows the blood vessel diameter. Statistical significance was calculated using the one-way ANOVA test (**$p \leq 0.01$, *** $p \leq 0.001$, **** $p \leq 0.0001$ treated Sam0.7 vs. Sam_cntrl group).

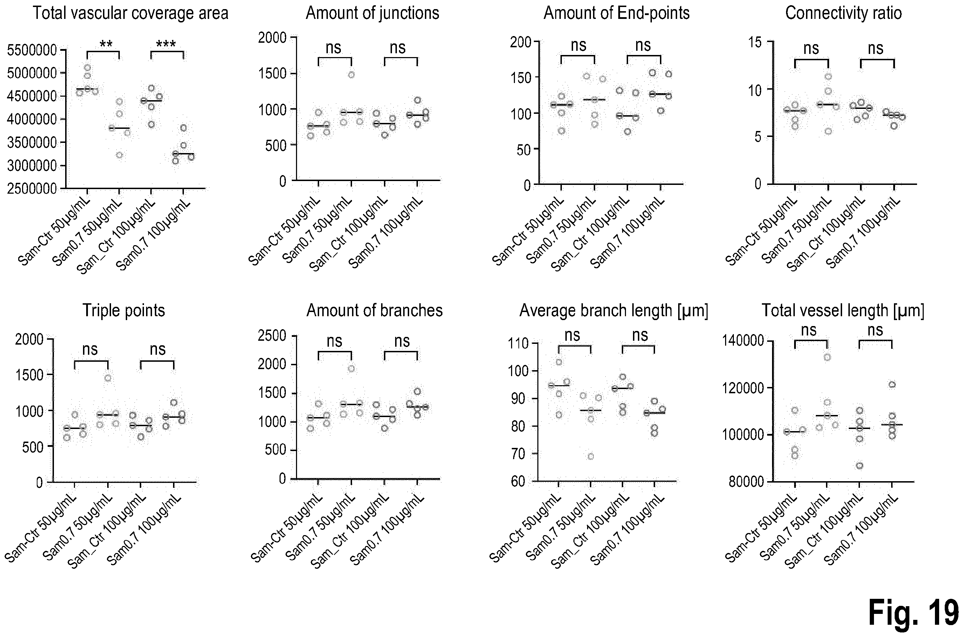

Fig. 19: The effect of the designed binder Sam0.7 on microvasculature formation *in vitro.* Quantification of microvasculature parameters of capillaries treated with Sam0.7 or Sam_cntrl (as a negative control). The connectivity ratio is defined as the ratio of junctions to end-points. Statistical significance was calculated using the one-way ANOVA test (** $p \leq 0.01$, *** $p \leq 0.001$ treated Sam0.7 vs. Sam_cntrl group).

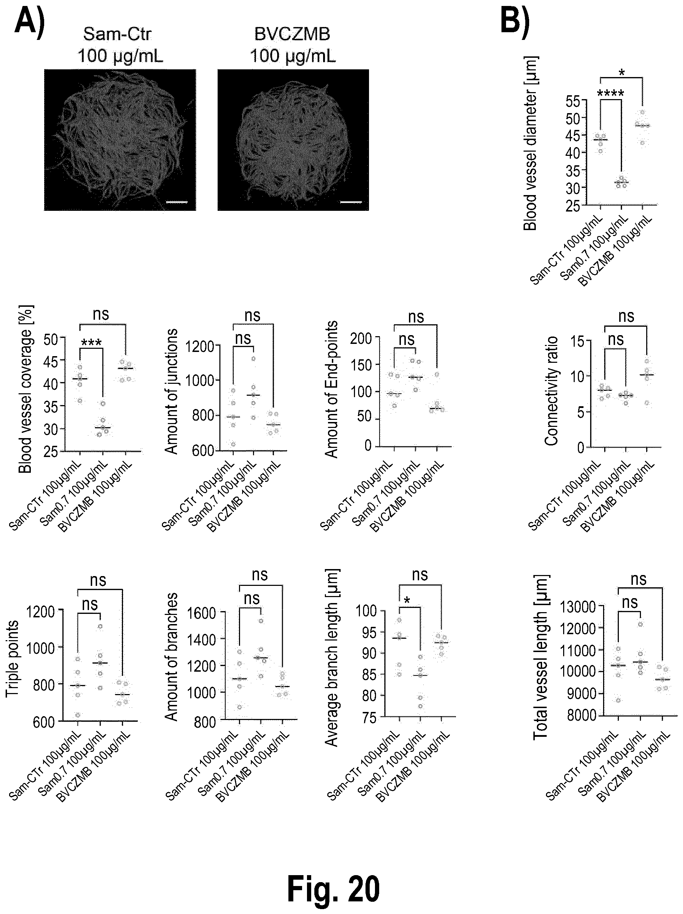

Fig. 20: The effect of Bevacizumab on the microvasculature formation *in vitro.* (A) Representative images showing *in vitro* microvasculature formation in the presence of Bevacizumab or Sam_cntrl as a negative control at the same working concentrations (100 $\mu$g/mL). The scale bar is 500 $\mu$m. (B) Quantitative analysis of microvasculature formation. Statistical significance was calculated using the one-way ANOVA test (* $p \leq 0.05$, *** $p \leq 0.001$, **** $p \leq 0.0001$ treated Sam0.7 or Bevacizumab vs. Sam_cntrl group).

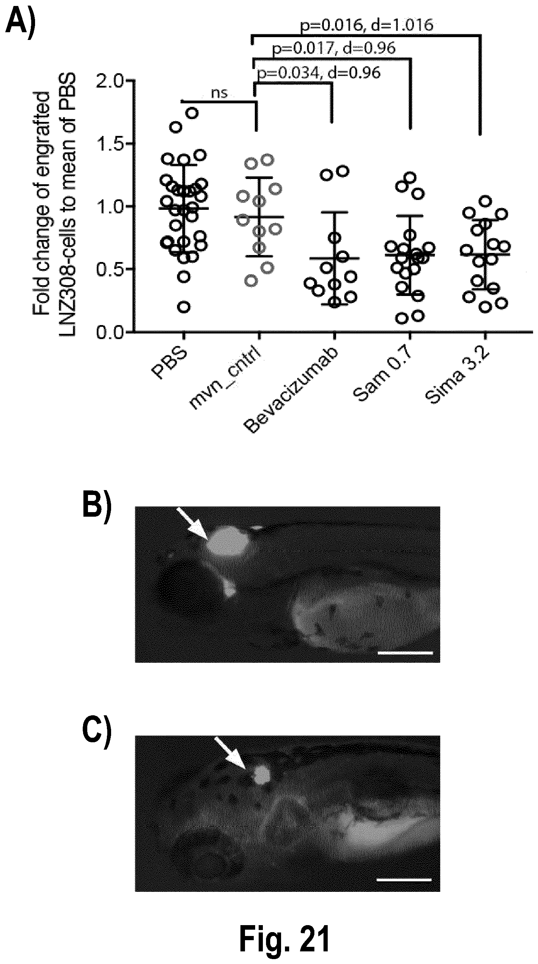

Fig. 21: The designed binders show *in vivo* activity in zebrafish xenograft. (A) Quantification of the engrafted LNZ308-GFP glioma cells in zebrafish embryos that were injected with PBS, inactive protein (mvn_cntrl) as negative control, Bevacizumab as positive control, Sam 0.7, or Sima 3.2. Each dot indicates one embryo. p-value was calculated by Mann Whitney two tailed test. d - Cohen's d value. (B) Representative zebrafish xenograft treated with PBS. (C) Representative zebrafish xenograft treated with Sam 0.7. Arrowheads indicate transplanted LNZ308-GFP cells in the brain. The scale bar is 200$\mu$m.

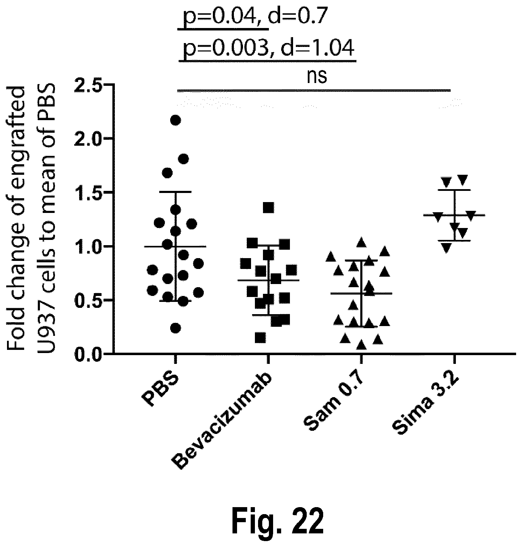

Fig. 22: Evaluation of inhibitory activity of the designs in leukemia zebrafish xenograft. Quantification of the engrafted U937-GFP leukemia cells in zebrafish embryos that were injected with PBS, Bevacizumab as positive control, Sam 0.7, or Sima 3.2. Each dot indicates one embryo. p-value was calculated by Mann Whitney two tailed test. d-Cohen's d value.

EMBODIMENTS

1. Results

*Sarting point and strategy for de novo design of epitope-targeted binders*

Protein-protein interactions play a central role in all biological processes, thus the ability to design and manipulate such interactions is of immense value for basic and applied research. Particularly, the design of on-demand binders against a predefined target epitope greatly broadens the range of accessible therapeutic applications. Such epitope targeting, in addition to the control over the binder's shape and biophysical properties, can be achieved through computational protein design. Previous studies have demonstrated successful protein binder design based on information derived from natural complex structures, where key interaction residues were incorporated into new, geometrically-accommodating scaffolds. Alternatively, *de novo* backbones have been built around natural binding motifs. Although these template-based approaches are rapidly advancing, they cover only a narrow range of targetable surfaces and are not applicable for novel interface design.

Meanwhile, template-free binder design - i.e., without using structural information from known binders - remains an outstanding challenge. Recent studies contributed towards de novo binder design. Cao et al. applied the rotamer interaction field (RIF) docking method to generate binders against SARS-CoV-2 spike protein, and twelve other diverse target proteins; see Cao, L., et al., De novo design of picomolar SARS-CoV-2 miniprotein inhibitors. Science, 2020. 370(6515): p. 426-431, and Cao, L., et al., Design of protein-binding proteins from the target structure alone. Nature, 2022. 605(7910): p. 551-560. An alternative geometric deep learning tool, MaSIF-seed, has been proposed recently to identify structural motifs able to engage specific epitopes based on geometrical and chemical complementarity. This tool enabled design of protein binders for three different therapeutically relevant targets - SARS-CoV-2 spike, PD-1, and PD-L1; Gainza, P., et al., De novo design of site-specific protein interactions with learned surface fingerprints. Nature, 2023, 617: p. 176-184. However, all these approaches suffer from low success rates (below 1 %), and thus have to rely on the screening of thousands of designs. This motivates the development and evaluation of further strategies for *de novo* design of protein binders.

An ideal binder must possess maximum binding affinity and specificity, while being stable in the presence and absence of its target. This is a formidable challenge since improving one of these properties often comes at the cost of the other two. Moreover, binder design requires the simultaneous optimization of shape complementarity, polar and hydrophobic interactions at the interface, and the overall solvation energy of the bound and free forms.

The inventors explored a tiered approach that first prioritizes the maximization of shape complementarity, then follows to optimize and refine the other designable parameters. The first step of identifying complementary binding scaffolds is achieved through high throughput docking of a database of protein structures against the target epitope. To this end, the inventors devised an ultra-fast algorithm (Highly Efficient Complementarity Testing by Obverse Residuals, HECTOR), which enables the evaluation of surface complementarity through a vectorized, lower dimensional surface representation. The second step involves the sequence design and *in silico*-filtering of the binding candidates (Fig. 1).

The utility of the inventors' pipeline was demonstrated by designing binders targeting the receptor-binding site of vascular endothelial growth factor (VEGF), with the aim of inhibiting its activity. VEGF is a key regulator of normal and pathological angiogenesis, and is a validated target for anti-cancer, cardiovascular, and ophthalmic therapies. VEGF forms a 2:2 complex with the ectodomain of its receptor (VEGFR), which triggers activation of the intracellular receptor tyrosine kinase domain and the subsequent signaling cascades (Fig. 2A). Current anti-VEGF therapies include both small molecules and protein-based inhibitors. Small molecules act by inhibiting the VEGFR intracellular kinase domain, however, they are less specific and more toxic than the available protein-based therapies. On the other hand, protein-based inhibitors, which block the VEGF:VEGFR interaction, are large, complex molecules that are post-translationally modified. Therefore, the inventors aimed to design inhibitors based on simple, single-domain scaffolds. Having tested only 16 candidates, the inventors identified proteins that showed strong VEGF binding, anti-VEGF activity in cell-based and *in vivo* assays, highlighting their therapeutic potential.

The first step of the inventors' pipeline aims at identifying binder scaffolds with highly complementary surface patches to the query epitope. Docking a query epitope against a large database of template structures is, however, a computationally demanding task, particularly given the rotational and translational degrees of freedom associated with the docking problem. In order to achieve ultra-fast steric docking evaluation for two (query and subject) surface patches, the inventors derived a surface fingerprint. The HECTOR fingerprint (Highly Efficient Complementarity Testing by Obverse Residuals) combines the two advantageous properties of invertibility and compression. Through the first property, a fingerprint describing a query surface patch can be inverted through a single transformation to describe the ideally complementary surface patch (Fig. 3A). Consequently, maximizing the similarity of the inverted query fingerprint to a subject fingerprint becomes a simple and straightforward optimization, that effectively maximizes the complementarity between the underlying query and subject surfaces (Fig. 3B). The second property relates to the lower dimensional projection of a three-dimensional surface patch by a two-dimensional matrix. While this compression is lossy, it is necessary to achieve rotational invariance of a fingerprint (Materials and methods).

In this layout, a protein structure database is used as a source of scaffolds. Specifically, here the inventors used a snapshot of high-resolution X-ray structures from the Protein Data Bank (PDB). The dot-surfaces of all-atom representations of these structures are derived (adding the coordinates of missing atoms) and stored. Overlapping surface patches are then extracted from these dot-surfaces, which are then forward-mapped to yield a database of fingerprints. While this large-scale mapping tessellation and fingerprinting can be computationally expensive, it is performed only once. A query epitope (one or more) undergoes the same procedure, however, the z-coordinate is obversely scaled (i.e. $k = -1$) to obtain the inverse fingerprint (Materials and methods). The complementarity between the query and subject surface patches is evaluated as an R-factor that quantifies the dissimilarity between the two corresponding matrices (i.e. fingerprints). Therefore, lower R-factor values indicate higher complementarity (Fig. 4). Particularly, identifying clusters of query and subject surface patches that are spatially proximal can be used to identify putative binding sites (Fig. 5, 6). The R-factor calculation could be efficiently implemented in a vectorized form on graphics processing units (GPUs), where a single evaluation fell within sub-microsecond timeframe. This allowed the inventors to carry out large-scale, shape-based docking at a high sampling granularity, where the overlapping surface patch centers were on average 1.5 Å apart.

Upon identifying scaffolds that harbor highly complementary surface patches, complementary patches would provide distance restraints for docked pose generation and refinement. These highly complementary complex models would serve as input for one-sided interface design, where the scaffold residues at the binding interface are designed to optimize the interactions with the query epitope. To more rigorously rank the designed complexes, two types of molecular dynamics (MD) simulations were used to predict the configurational stability and binding energy of the designed complexes.

*Computational design of the VEGF binders*

Using described strategy, the inventors sought to design proteins able to prevent activation of the VEGFR by quenching the receptor-binding site of VEGF (Fig. 2A). In order to find scaffolds of complementary shape, the inventors applied the HECTOR software to two surface patches on the VEGF binding site, which is structurally well characterized; Muller, Y.A., et al., Vascular endothelial growth factor: crystal structure and functional mapping of the kinase domain receptor binding site. Proc Natl Acad Sci USA, 1997. 94(14): p. 7192-7; Brozzo, M.S., et al., Thermodynamic and structural description of allosterically regulated VEGFR-2 dimerization. Blood, 2012. 119(7): p. 1781-8. These surface patches were inverse-mapped and docked against a library of fingerprints pre-computed from a high-resolution subset of PDB crystal structures. The inventors retrieved six HECTOR hits (Fig. 5) and locally docked them against VEGF using PatchDock; Schneidman-Duhovny, D., et al., PatchDock and SymmDock: servers for rigid and symmetric docking. Nucleic Acids Res, 2005. 33(Web Server issue): p. W363-7. The inventors selected two of these for further design: a bacterial ketosteroid isomerase (PDB: 1OH0) and a nitrophorin, hemoprotein of blood-feeding insects (PDB: 1PM1). Both templates showed a strong shape complementarity signal to the VEGF β-hairpin loop at their respective catalytic pockets. Both also possess much simpler architecture than published anti-VEGF antibodies and have molecular weights of 15 kDa and 20 kDa, respectively. The inventors named the designs utilizing the first scaffold Sam and those using the second one Sima (Fig. 2B).

The inventors then performed interface design by designing positions on the binder structure chosen automatically based on their proximity from the target surface (Cα distance ≤ 8 Å). Sequence and conformers sampling was done using the RosettaScripts framework; Fleishman, S.J., et al., RosettaScripts: a scripting language interface to the Rosetta macromolecular modeling suite. PLoS One, 2011. 6(6): p. e20161. The interface design was followed by two MD scoring steps, i.e. temperature-accelerated MD and potential of mean force calculations from non-equilibrium steered MD. The inventors selected eight of the best-scoring Sam designs (Sam0.1 - Sam0.8) for experimental validation. The final sequences (Table 1) have 19 to 24 mutations compared to the starting template. For Sima proteins, the inventors chose four candidates for testing where the native disulfide bonds of the scaffold were retained (Sima1.1 - Sima4.1) and four candidates where the disulfide bonds were eliminated (Sima1.2 - Sima4.2). The final Sima sequences (Table 1) contain 25 to 28 mutations compared to the scaffold.

Table 1: Protein sequences of Sam/Sima templates and the designed proteins

| Name | Sequence | Global sequence identity with the initial template | Molecular weight (Da) |
|---|---|---|---|
| PDB 1OH0 | LPTAQEVQGLMARYIELVDVGDIEAIVQMYADDATVED PFGQPPIHGREQIAAFYRQGLGGGKVRACLTGPVRAS HNGCGAMPFRVEMVWNGQPCALDBVIDVMRFDEHG RIQTMQAYWSEVNLSVREP (SEQ ID NO: 19) | - | 14162 |
| Sam0.1 | LPTAQEVQGLMARYIELMDVGDIEAIVQMYADDATVEA PFGAPPIHGRERIAYFYRRMLGGGIARATLTGPVRASH NGTGAMPFRVEFVFNGQPYAMDVRVEMRFDEHGRIQ TMQAYWSWVNLSVREP (SEQ ID NO: 5) | 109/128 (85 %) | 14437 |
| Sam0.2 | LPTAEEVQKLMARYIELMDVGDIEAIVQMYADDATVEA PFGAPPIHGRERIAYFYRRMLGEGIARATLTGPVRASH NGTGAMPFRVEYVFNGQPYAMDVRVEMRFDEHGRIQ TMQAYWSEVNDSVRE (SEQ ID NO: 6) | 109/128 (83 %) | 14543 |

(continued)

| Name | Sequence | Global sequence identity with the initial template | Molecular weight (Da) |
|------|----------|---------------------------------------------------|----------------------|
| Sam0.3 | LPTAEEVQKLMARYIELMDKGDIEAIVQMYADDATVEA PFGAPPIHGRERIAYFYRRMLGEGIARATLTGPVRASH NGTGAMPFRVEFVFNGQPYAMDVRVEMRFDEHGRIQ TMQAYWSWVNLSVREP (SEQ ID NO: 7) | 105/128 (82 %) | 14611 |
| Sam0.4 | LPTAEEVQKLMARYIELLDVGDIEAIVQMYADDATVEA PFGSPPIHGRERIAYFYRRMLGEGIARATLTGPVRASH NGTGAMPFRVEYVLNGQPVAMDIRVEMRFDEHGRIQ TMQAYWSWVNVSVREP (SEQ ID NO: 8) | 104/128 (81 %) | 14498 |
| Sam0.5 | LPTAEEVQKLMARYIELMDVGDIEAIVQMYADDATVEA PFGAPPIHGRERIAYFYRRMLGGGIARATLTGPVRASH NGTGAMPFRVEYVFNGQPYAMDVRVEMRFDEHGRIQ TMQAYWSWVNLSVREP (SEQ ID NO: 9) | 107/128 (84 %) | 14526 |
| Sam0.6 | LPTAEEVQKLMARYIELMDVGDIEAIVQMYADDATVEA PFGAPPIHGRERIAYFYRRMLGGGIARATLTGPVRASH NGTGAMPFRVEFVFNGQPFAMDVRVEMRFDEHGRIQ TMQAYWSWVNLSVREP (SEQ ID NO: 10) | 107/128 (84 %) | 14494 |
| Sam0.7 | LPTAEEVQKLMARYIELMDVGDIEAIVQMYADDATVEA PFGAPPIHGRERIAYFYRRMLGGGIARATLTGPVRASH NGTGAMPFRVEYVFNGQPFAMDVRVEMRFDEHGRIQ TMQAYWDWVNLSVREP (SEQ ID ON: 3) | 106/128 (83 %) | 14538 |
| Sam0.8 | LPTAEEVQKLMARYIELMDVGDIEAIVQMYADDATVEA PFGAPPIHGRERIAYFYRRMLGGGIARATLTGPVRASH NGTGAMPFRVEYVFNGQPYAMDVRVEMRFDEHGRIQ TMQAYWSWVNISVREP(SEQ ID NO: 11) | 106/128 (83 %) | 14526 |
| PDB 1PM1 | DCSTNISPKQGLDKAKYFSGKWYVTHFLDKDPQVTDQ YCSSFTPRESDGTVKEALYHYNANKKTSFYNIGEGKL ESSGLQYTAKYKTVDKKKAVLKEADEKNSYTLTVLEAD DSSALVHICVREGSKDLGDVYTVLTHQKDAEPSAKVK SAVTQAGLQLSQFVGTKDLGCQYDDQFTSL (SEQ ID NO: 20 | - | 19894 |
| Sima1.1 | DCSTNISPKQGLDKAKYFSGKWYLTHVLIKDPVAVTQF CSSFTPRESDGTVKEAIYVYLAIKKTSEYAIGEGKLESS GLQYTATFKTVDKKKAVLKEWDERYSYTITVLEADDSS ALTHVCTREGSKDYGDYYHVLTHQKDAEPSAKVKSAV TQAGLQLSQFVGTKDLGCQYDDQFTSL (SEQ ID NO: 12) | 153/179 (85 %) | 19957 |
| Sima2.1 | DCSTNISPKQGLDKAKYFSGKWYLTHVLIKDPVAVTQF CSSFTPRESDGTVKEAIYVYLAIKKTSEYAIGEGKLESS GLQYTATFKTVDKKKAVLKEWDERYSYTITVLEADDSS ALTHVCTREGSKDYGDYYMVLTHQKDAEPSAKVKSA VTQAGLQLSQFVGTKDLGCQYDDQFTSL (SEQ ID NO: 13) | 153/179 (85 %) | 19951 |

(continued)

| Name | Sequence | Global sequence identity with the initial template | Molecular weight (Da) |
|---|---|---|---|
| Sima3.1 | DCSTNISPKQGLDKAKYFSGKWYLTHVLIKDPKIVSQF CASFTPRESDGTVKIAVYLYLAIKKTSDYAIGEGKLESS GLQYTATSKVVDKKKAVLKELDERHSYTVTVLEADDS SALTHICVREGSKDYGDYYLVLTHQKDAEPSAKVKSA VTQAGLQLSQFVGTKDLGCQYDDQFTSL (SEQ ID NO: 14) | 152/179 (85 %) | 19781 |
| Sima4.1 | DCSTNISPKQGLDKAKYFSGKWYLTHVLIKDPVAVTQF CSSFTPRESDGTVKVAIYVYLAIKKTSEYAIGEGKLESS GLQYTATFKTVDKKKAVLKEFDERHSYTITVLEADDSS ALVHVCVREGSKDYGDYYNVLTHQKDAEPSAKVKSA VTQAGLQLSQFVGTKDLGCQYDDQFTSL (SEQ ID NO: 15) | 154/179 (86 %) | 19835 |
| Sima1.2 | STNISPKQGLDKAKYFSGKWYLTHVLIKDPVAVTQFCS SFTPRESDGTVKEAIYVYLAIKKTSEYAIGEGKLESSGL QYTATFKTVDKKKAVLKEWDERYSYTITVLEADDSSAL THVTTREGSKDYGDYYHVLTHQKDAEPSAKVKSAVTQ AGLQLSQFVGTKDLGCQYDDQFTSL (SEQ ID NO: 16) | 150/177 (85 %) | 19737 |
| Sima2.2 | STNISPKQGLDKAKYFSGKWYLTHVLIKDPVAVTQFCS SFTPRESDGTVKEAIYVYLAIKKTSEYAIGEGKLESSGL QYTATFKTVDKKKAVLKEWDERYSYTITVLEADDSSAL THVTTREGSKDYGDYYMVLTHQKDAEPSAKVKSAVT QAGLQLSQFVGTKDLGCQYDDQFTSL (SEQ ID NO: 17) | 150/177 (85 %) | 19731 |
| Sima3.2 | STNISPKQGLDKAKYFSGKWYLTHVLIKDPKIVSQFCA SFTPRESDGTVKIAVYLYLAIKKTSDYAIGEGKLESSGL QYTATSKVVDKKKAVLKELDERHSYTVTVLEADDSSAL THITVREGSKDYGDYYLVLTHQKDAEPSAKVKSAVTQ AGLQLSQFVGTKDLGCQYDDQFTSL SEQ ID NO: 4) | 149/177 (84 %) | 19561 |
| Sima4.2 | STNISPKQGLDKAKYFSGKWYLTHVLIKDPVAVTQFCS SFTPRESDGTVKVAIYVYLAIKKTSEYAIGEGKLESSGL QYTATFKTVDKKKAVLKEFDERHSYTITVLEADDSSAL VHVTVREGSKDYGDYYNVLTHQKDAEPSAKVKSAVT QAGLQLSQFVGTKDLGCQYDDQFTSL (SEQ ID NO: 18) | 151/177 (85 %) | 19615 |

*Biophysical characterization of the designed VEGF inhibitors*

As a first experimental step, the inventors expressed the chosen designs in *E. coli* and double-purified them, in most cases, with good yields (not shown). The inventors then tested their oligomeric state with analytical size exclusion chromatography, revealing all the Sam designs to be monomeric and the Sima designs to exist as monomeric, dimeric, and tetrameric forms (Fig. 7). Based on expression yield and preliminarily estimated binding to VEGF (Fig. 8, Fig. 9), the inventors chose the best-performing design from each group for full characterization; Sam0.7 and Sima3.2.

The inventors evaluated the thermostability and aggregation propensity of the proteins adopting nano differential scanning fluorimetry and backreflection measurements. Sam0.7 and Sima3.2 have similar melting temperatures (63 °C and 65 °C, respectively, Fig. 10A). However, light scattering thermograms revealed that Sima3.2 has a higher propensity for aggregation with an onset temperature of around 40 °C, compared to 60 °C for Sam0.7 (Fig. 10B).

To characterize the kinetics and affinity of interactions between the designed proteins and VEGF, the inventors performed surface plasmon resonance-based binding assays. Analysis of the kinetics across the injection dilution series, assuming 1:1 binding, resulted in dissociation constants (Kd) of 190 nM and 14 nM for Sam0.7 and Sima3.2, respectively.

The higher affinity of Sima3.2 could be ascribed to its slower dissociation rate, compared to Sam0.7 (Fig. 10C, Fig. 11).

*Structural characterization of the designs*

To evaluate the accuracy of the computational design, the inventors sought to determine experimental structures of the designed binders. The inventors obtained crystal structures for the unbound Sam0.2 and Sam0.7 proteins. Alignment of the experimental structures to the corresponding design models showed that there is an atomic-level agreement with overall backbone $C\alpha$ root mean square deviation (RMSD) of 1.8 Å for Sam0.2 and 1.7 Å for Sam0.7 (Fig. 12, Fig. 13, Table 2).

Table 2: Crystallographic Data Collection and Refinement Statistics. [a] Values in parenthesis refer to the highest-resolution shell.

| Structure | Sam0.2 | Sam0.7 |
|---|---|---|
| **Data collection** | | |
| Space group | P6s22 | P6s22 |
| Cell parameters a, b, c (Å) | *77.81, 77.81, 78.16* | *40.55, 40.55, 253.12* |
| Wavelength (Å) | 1.000 | 1.000 |
| Resolution limits (Å) | 39.08-2.65 (2.81-2.65) | 35.12.1.80 (1.91-1.80) |
| Unique reflections | 4410 (686) | 12500 (1934) |
| Completeness (%) | 100 (100) | 100 (99.9) |
| Redundancy | 36.70 (40.09) | 32.77 (34.94) |
| I/σI | 31.5 (1.36) | 19.0 (1.65) |
| Rmerge (%) | 8.4 (294.7) | 10.3 (162.3) |
| CC(1/2) | 100 (72.7) | 99.9 (96.5) |
| **Refinement** | | |
| Resolution limits (Å) | 39.08-2.65 | 35.12.1.80 |
| Rcryst (%) | 25.7 | 25.7 |
| Rfree (%) | 29.8 | 29.6 |
| Protein molecules/ asymmetric unit | 1 | 1 |
| Mean B value (Å) | 110.5 | 50.5 |
| **PDB code** | **8BL5** | **8BL9** |

Further analysis of the per residue deviations revealed that regions with the highest mismatch ($C\alpha$ RMSD per residue > 3 Å) correspond to the outer rim of the Sam binding site (e.g., R56 - M58, A64, F95, A96, D119 - V121 for Sam0.7, Fig. 12B). The inventors ascribe this to the opened and closed conformations of Sam in its bound and unbound states, respectively. While designed coordinates of Sam were derived from its complex with VEGF (MD-relaxed structure, Materials and methods), the crystal structures represent isolated Sam monomers.

To further characterize the Sam binding mode, the inventors expressed $^{13}$C, $^{15}$N-labelled protein for NMR spectroscopy. The $^{15}$N-HSQC fingerprint of the protein is well dispersed, indicative of a folded protein. However, experiments acquired toward sequential assignment of the protein showed multiple peaks for several residues (e.g., G60 and A61, Fig. 14). This is indicative of conformational exchange that is slow on NMR chemical shift timescales; i.e. milliseconds. For other residues $^{15}$N-HSQC were not assigned due to low intensities or were missing altogether. These residues broadly coincide with loops on the parent structure. Despite this, sufficient residues were assigned to allow mapping of the VEGF binding epitope. The inventors titrated labelled Sam with unlabeled VEGF and recorded $^{15}$N-HSQC at two VEGF concentrations. Given the measured affinity of Sam for VEGF, the inventors should expect slow exchange between free and bound species at the concentrations used for the NMR samples; i.e. new peaks corresponding to bound Sam should appear as VEGF concentrations increase, while peaks for free Sam disappear. However, these observations are hampered by the large size of the Sam/VEGF complex, together with the low intensity of many free VEGF peaks in the region of the binding epitope. Nevertheless, peaks for some residues show the expected behavior (Fig. 14) and map broadly to the designed epitope. Other peaks show gradual shifts on titration of VEGF, which could be the result of weak binding to a secondary epitope.

*In vitro and in vivo evaluation of anti-VEGF activity*

VEGF secreted by cancer cells provides mitogenic and survival stimuli for endothelial cells, leading to the formation of new blood vessels, and in turn, tumor expansion. In order to assess the inhibitory capability of the designed proteins, the inventors tested their anti-angiogenic effect on VEGF-responsive primary human endothelial cells, HUVEC. Indeed, the addition of either Sam0.7 or Sima3.2 into a culture medium in the low micromolar range significantly decreased VEGF-induced cell survival (Fig. 15A).

In addition to the regulation of angiogenesis, autocrine and para-crine VEGF/VEGFR signaling in tumor cells contributes to cancer cell proliferation, survival, induction of the epithelial-mesenchymal transition, and metastasis. For example, autocrine expression of VEGF has been reported to play a role in hematopoietic malignancies by stimulating growth and migration of leukemic cells. The inventors, therefore, decided to evaluate the efficacy of their designs on the proliferation of the acute myeloid leukemia cell line, U937, that is sensitive to VEGF. Treatment of U937 cells with increasing concentrations of the designed binders reduced cell proliferation down to 60 % in case of Sam0.7 and down to 50 % in case of Sima3.2 (Fig. 15B). Further analysis revealed half-maximal inhibitory concentrations (IC50) to be 8.0 $\mu$g/ml (533 nM) and 3.6 $\mu$g/ml (180 nM) for Sam0.7 and Sima3.2, respectively (Fig. 16). To test whether detected anti-VEGF activity of the inventors' designs is mediated by residues introduced during interface design step, they performed U937 proliferation assay for the initial design templates of Sam and Sima (Sam_cntrl and Sima_cntrl, respectively). No inhibitory effect was observed (Fig. 15B). Additionally, the inventors evaluated the effect of the designed binders on the human embryonic kidney cell line, HEK293T, that does not express endogenous VEGFR. The inventors observe a mild reduction in cell growth only after treatment of HEK293T cells with the highest concentration of Sam0.7 (100 $\mu$g/ml), demonstrating that their designs specifically target VEGF-dependent cells (Fig. 17).

After confirming the inhibitory effect of Sam0.7 on VEGF-dependent cell types, the inventors proceeded to evaluate its anti-angiogenetic activity, using an *in vitro* microvascularformation model. In this experiment, a self-assembled 3D microvasculature was created by co-culturing hiPSC-derived endothelial cells and pericytes on a 3D fibrin gel. The microvasculature was treated with either Sam0.7 or Sam_cntrl (Fig. 18A). Treatment with Sam0.7 at both tested concentrations (50 $\mu$g/ml and 100 $\mu$g/ml) significantly reduced the angiogenesis *in vitro* in a dose-dependent manner (Fig. 18B). Specifically, the microvasculature treated with Sam0.7 exhibited lower area coverage and thinner blood vessels compared to the microvasculature treated with Sam_cntrl (Fig. 18C). Other microvascular parameters, such as connectivity, total vessel length, amount of branches, and others remained unchanged (Fig. 19). Moreover, Sam0.7 showed stronger effect on the microvasculature formation then 100 $\mu$g/ml bevacizumab - a highly potent antibody-based VEGF inhibitor that is approved for the treatment of several cancer types (Fig. 20).

To further evaluate the pharmacological potential of the designs, the inventors studied their effects on the VEGF-dependent solid tumor and blood cancer cells *in vivo.* Injection of 3.5 mg/ml (4 nl) of Sam0.7 into the brain of zebrafish embryos at 33 hours post fertilization (hpf) led to a decrease in the number of transplanted LNZ308 glioma cells after 2 days treatment (Fig. 21) at a comparable level to injection of 25 mg/ml of bevacizumab. A similar effect was observed when the design was tested on a leukemic cell line. Injection of Sam0.7 in the bloodstream of zebrafish embryos, where the transplanted leukemia cells, U937, were engrafted (Fig. 22), led to their reduction.

Treatment of zebrafish embryos with 2.4 mg/ml (4 nl) of Sima3.2 over 2 days had a significant inhibiting effect on LNZ308 cells when it was injected directly into the brain (Fig. 21). Injection of Sima3.2 into the bloodstream of 33 hpf embryos did not reduce U937 cells and showed high lethality rate as opposed to PBS control and Sam0.7 injections (Fig. 22). This the inventors attribute to the higher aggregation propensity of Sima3.2 in solution compared to Sam0.7.

<u>2. Material and methods</u>

*Computational design of de novo binders*

The surface mapping and steric docking step begins by surface tessellation of the entire protein structure, to yield a dot-surface $S_c$ of the molecular surface in the Cartesian coordinate system The associated vector field describing the outward-pointing surface normal vector at each surface dot $S_n$ was derived. The position vector and surface normal at each dot would thus define the local origin and z-axis at each local surface patch. The protein's dot-surface was split into a number of overlapping surface patches with an average origin-to-origin spacing of 1.5 Å, where each surface patch

$$Fi = \left\{ \mathbf{s}_c \middle| \mathbf{s}_c \in S_c, \quad \frac{\left\| (\mathbf{s}_c - \mathbf{s}_{c_i}) \times \mathbf{s}_{n_i} \right\|}{\left\| \mathbf{s}_{n_i} \right\|} < R_{max} \quad \left| (\mathbf{s}_{ci} + \mathbf{s}_{ni}) \cdot (\mathbf{s}_c) \right| < \frac{H_{max}}{2} \right\}$$

is a set of the Cartesian coordinates (x, y, z) of surface dot $\mathbf{s}_c$ that lie within a maximum distance $R_{max}$ from the surface normal $\mathbf{s}_{ni}$ of the $i^{th}$ dot $\mathbf{s}_{ci}$ (i.e. the center of the patch), and are within half the maximum axial distance (along the surface normal vector $\mathbf{s}_n$). These individual surface patches contain their frame of reference where $\mathbf{s}_{ci}$ is the origin and $\mathbf{s}_{ni}$ is the local z-axis to be projected into a cylindrical coordinate system to yield a forward or inverse 3D map $G_i$ according to:

$$T_p : f(x, y, z) \rightarrow g(r, \theta, l) := \begin{bmatrix} \sqrt{x^2 + y^2} \\ 1 \Big/ \tan\left(\frac{y}{x}\right) \\ k \cdot z \end{bmatrix} \qquad (1)$$

A projected $G_i$ can be mapped in either a forward ($G+_i$) or an obverse ($G-_i$) direction, depending on the value of

$$k = \begin{cases} +1 & forward \\ -1 & obverse \end{cases}$$

to describe a forward- or obverse-mapped surface patch. At this stage, projected $G\pm_i$ patches are translation-invariant, and rotation-invariant along two principal planes; i.e. the 3D projections are only sensitive to alignments along the $\theta$ dimension. Therefore, the indefinite integral across the $\theta$ dimension (eq. 2): i) reduces the dimensionality of the 3D projections into compressed 2D density projects; albeit at the loss of some information, ii) renders the 2D maps rotationally-invariant, and iii) possess constant dimensions.

$$T_r : g\pm(r, \theta, l) \rightarrow h\pm(r, l) := \int g\pm(r, \theta, l) d\theta \qquad (2)$$

Lastly, to decrease the sensitivity to minor resolution errors, a bilinear interpolation step (eq. 3) is applied to $H\pm_i$ to blur slight deviations in surface-dot densities, and allow for a degree of conformational insensitivity:

$$T_r : h_{\pm}(r, l) \rightarrow j_{\pm}(r, l) := \sum_{i=0}^{1} \sum_{j=0}^{1} a_{ij} x^i y^j \qquad (3)$$

This yields the final form of the fingerprint $j\pm(r, l)$ that can be directly used for complementarity matching by calculating an R-factor $R_{s,q}$ that best describes the normalized differences between two matrices describing the forward- and obverse-mapped query and subject maps, respectively:

$$R_{s,q} = \frac{\sum \sqrt{\left\| j_{s,\pm}(r,l) - j_{q,\mp}(r,l) \right\|}}{\sum \sqrt{\left\| j_{s,\pm}(r,l) \right\|}} \qquad (4)$$

A 2018 Protein Data Bank snapshot was filtered down to the X-ray structures with resolution better than 2.0 Å, their hydrogen atoms coordinates were guessed, and the structures dot-surfaces were derived. These were forward-mapped as subject patches $j_{s,}+(r, l)$, where r covers radial distance range of [0,12] Å from the surface normal axis of the patch, and $l$ covers the axial distance range of [-6,6] Å. The final maps were composed of $30 \times 30$ bins, where the resolution is 0.4 Å.

Two receptor binding surface patches were used as query in a two-against-two search, which was bounded by a distance restraint with a tolerance of $\pm 3$ Å. The retrieved hits were docked against VEGF based on their surface complementarity using PatchDock; Schneidman-Duhovny, D., et al. (l.c.). For that, the residues corresponding to patches with low R-factor in both the hit and VEGF were set as binding residues and used as constraints for rigid body docking using default parameters, as set by the buildParamsToOutfile.pl PatchDock utility. For each job, the top few poses were selected and further refined by using short, low-temperature molecular dynamics simulations. These simulations were run with generalized Born implicit solvent, where the temperature was set to 250 K using a Langevin thermostat through the NAMD2 software; Kalé, L., et al., NAMD2: Greater Scalability for Parallel Molecular Dynamics. Journal of Computational Physics, 1999. 151(1): p. 283-312.

For the one-sided interface design stage, a design protocol was implemented in RosettaScripts (Fleishman, S.J., et al., *l.c.*) that deploys multiple design movers interlaced with backbone movers. The movers were bundled within two main iterative generic Monte Carlo sampling stages, which implemented with interface $\Delta\Delta G$ and packstat (Sheffler, W. and D. Baker, RosettaHoles: rapid assessment of protein core packing for structure prediction, refinement, design, and validation. Protein Sci, 2009. 18(1): p. 229-39) filters for scoring. This design protocol was run for 100 instances per input decoy, and the entire procedure was performed in 4-6 successive design rounds. The top few thousand decoys were clustered to unique sequences and were further filtered using two-stage molecular dynamics ranking. The first stage filtered the designs using serial tempering simulations in implicit solvent between cool and hot basins of 250 K and 370 K for 6 and 8 ps, respectively. This was conducted for a total of 60 cycles separated by 100 steps of conjugate gradient minimization per simulation, for 3 replicas per decoy. The conformational homogeneity across the cycles and replicas were quantified for each decoy as a function of the all-against-all RMSD (Skokowa, J., et al., A topological refactoring design strategy yields highly stable granulopoietic proteins. Nature Communications, 2022. 13(1): p. 2948). This reduced the decoys to a few hundred candidates that were further filtered by a second stage of steered molecular dynamics in explicit solvent under constant pressure and temperature. In the latter stage, the binder's backbone atoms were fixed using harmonic restraints along the z-dimension, and VEGF was pulled along the z-dimension at a fixed velocity of 2 Å/ns using a force constant of 50 kcal/mol·Å. The estimated work of dissociation was derived from the potential of mean force as previously described (ElGamacy, M., et al., An Interface-Driven Design Strategy Yields a Novel, Corrugated Protein Architecture. ACS Synth Biol, 2018. 7(9): p. 2226-2235; ElGamacy, M., M. Coles, and A. Lupas, Asymmetric protein design from conserved supersecondary structures. J Struct Biol, 2018. 204(3): p. 380-387).

*Bacterial protein expression and purification*

Synthetic genes encoding the human VEGF165 (Gly34-Asp135) and the designs were cloned into the pET28a(+) expression vector between the Ndel and Xhol cloning sites in-frame with a thrombin cleavage site and an N-terminal poly-histidine purification tag (General Biosystems, Inc.; Synbio Technologies, Inc.). Plasmids were transformed into chemically competent *E. coli* BL21(DE3) using the heat shock method. Transformed cells were grown in LB medium supplemented with 40 $\mu$g/ml kanamycin at 37 °C. At OD600 of 0.6-1.0, cells were induced with 1mM IPTG and incubated overnight at 25°C for protein expression. For purification of all Sam designs and Sima3.2, cells were harvested by centrifugation at 5000 g at 4°C for 20 min and lysed in 25 ml of lysis buffer (1M guanidinium chloride, 100 mM NaCl, 50mM Tris-HCl pH 8.0) supplemented with a tablet of the cOmplete, EDTA-free Protease Inhibitor Cocktail (Roche, 5056489001) and 3 mg of lyophilized DNase I (PanReac AppliChem, A3778) using a Branson Sonifier S-250 (Fisher Scientific). The lysate was cleared by centrifugation at 28000 g for 50 min and the supernatant was passed through a 0.45 $\mu$m filter (Millipore, SLHV033RS). The sample was applied to a 5 ml HisTrap HP column (Cytiva, GE17-5248-01). The running buffer was 150 mM NaCl, 30 mM Tris-HCl pH 8.0. After sequential washing the column with 30 ml of the running buffer supplemented with 0 or 50 mM imidazole, fractions were collected by linear gradient elution using 150 mM NaCl, 30 mM Tris-HCl pH 8.0, 500 mM imidazole buffer. In case of other Sima designs and human VEGF, proteins were extracted from the insoluble fraction of the cell pellet by stirring in a phosphate-buffered saline (PBS) with 5 M guanidinium chloride, and 25 mM DTTfor2 h at room temperature. The mixture was gradually diluted 5 times with PBS and cleared by centrifugation at 28000 g for 50 min. The proteins were purified from the filtered supernatants by immobilized metal-affinity chromatography (IMAC) as described above. The only difference is that the composition of the running buffer was 150 mM NaCl, 30 mM Tris-HCl pH 8.0, 1 M urea, 1.25 mM reduced glutathione, 0.25 mM oxidized glutathione. The eluted fractions containing the protein of interest were pooled, concentrated using 10 kDa MWCO centrifugal filters (Millipore, UFC901024), and further purified on a Superdex Increase 75 10/300 gel filtration column (Cytiva, 29148721) using PBS. Gel filtration fractions containing pure protein in the desired oligomeric state (monomeric fraction of Sam0.7, dimeric fraction of Sima3.2) were pooled, concentrated, and stored at -20 °C for subsequent analyses. Both IMAC and gel filtration steps were performed on an Äkta Pure chromatography system (Cytiva).

*Thermostability analysis*

Nanoscale differential scanning fluorimetry (nanoDSF) using Pro-metheus NT.48 (Nanotemper) was applied to evaluate thermostability of the designs. Capillaries (Nanotemper, PR-C002) were filled with 1 mg/ml protein samples in three replicates. Melting scan was performed across the temperature range from 20 °C to 90 °C with a temperature ramp of 1 °C/min. In addition to measuring the intrinsic fluorescence intensity ratio (350/330 nm), light intensity loss due to scattering (backreflection) was measured to detect protein aggregation.

*Isothermal Titration Calorimetry (ITC)*

To determine whether Sam designs bind to VEGF, ITC experiments were performed using MicroCal PEAQ-ITC (Malvern Panalytical). All samples were prepared in PBS. The cell was loaded with VEGF solution at concentrations from 80 to 500 $\mu$M for different measurements. The syringe was loaded with around 400 $\mu$M of Sam designs (375 $\mu$M of Sam0.2, 416 $\mu$M of Sam0.7). All measurements were carried out with the following parameters: 18 injections of 2 $\mu$l with 150 s between injections, reference power 41.9 $\mu$W, stirring speed 750 rpm, temperature 25 °C. Experimental data were fitted using MicroCal PEAQ-ITC Analysis Software. $K_d$ values were corrected taking into account N-value that describes active fraction of protein in the cell and in the syringe.

*Microscale thermophoresis (MST)*

To test ability of Sima designs to bind VEGF, MST measurements were performed on Monolith NT.115 (Nanotemper). Sima1.1, Sima3.1, and Si-ma3.2 were labeled with fluorescent dye using Protein Labeling Kit RED-NHS 2nd Generation (Nanotemper, MO-L011). For each experiment 16 samples were prepared with constant concentration of labeled Sima protein (100 nM of Sima1.1, 40 nM of Sima3.1, and 80 nM of Sima3.2) and decreasing concentrations of VEGF (2-fold dilutions from 12.5 $\mu$M to 0.4 nM for Sima1.1 measurements, from 64 $\mu$M to 2 nM for Sima3.1 measurements, and from 2 $\mu$M to 61 pM for Sima3.2 measurements). All dilutions were made in PBS supplemented with 0.1 % Pluronic®F-127 (Nanotemper). Measurements were done in Monolith NT.115 Premium capillaries (Nanotemper, MO-K025) at 25 °C. Dissociation constants ($K_d$) were derived by fitting normalized fluorescence values (cold region: -2 - -1 s, hot region: 4-6 s) against ligand concentrations.

*Surface plasmon resonance (SPR) binding assay*

Multi-cycle kinetics experiments were performed on a Biacore X100 system (GE Healthcare Life Sciences). Recombinant Human VEGF165 (rhVEGF) (R&D Systems, 293-VE-010/CF) was diluted to 50 $\mu$g/mL in 10 mM acetate buffer pH 5.0 and immobilized on the surface of a CM5 sensor chip (Cytiva, 29149604) using standard amine coupling chemistry. The designs were diluted in the running buffer (PBS with 0.05% v/v Tween-20). Analyses were conducted at 25°C at a flow rate of 10 $\mu$L/min. Five increasing concentrations of the sample solution (111 nM, 134 nM, 161 nM, 193 nM, 231 nM for Sam0.7; 44 nM, 66 nM, 100 nM, 148 nM, 222 nM for Sima3.2) were injected over the functionalized sensor chip surface for 60 s, followed by a 60 s dissociation with running buffer. At the end of each run, the sensor surface was regenerated with a 30 s injection of 50 mM NaOH at a flow rate of 10 $\mu$L/min. The reference responses and zero-concentration sensograms were subtracted from each dataset (double-referencing). Association rate ($k_a$), dissociation rate ($k_d$), and equilibrium dissociation ($K_d$) constants were obtained using a known linearization method.

*X-ray structure determination*

Crystallization screens were set up with a Mosquito robot (TTP Labtech) in 96-well plates at 21 °C, using 75 $\mu$l of reservoir solution and sitting drops containing 400 nl of reservoir and 400 nl of protein solution of either Sam0.2 or Sam0.7 at a concentration of 8 mg/ml. Within 7-14 days, crystals of Sam0.2 and Sam0.7 grew in a condition containing 20 % (w/v) PEG 3350 and 0.2 M KSCN. Crystals were cryoprotected through the addition of 15% PEG 400, flash cooled and stored in liquid nitrogen until data collection. Diffraction data were collected at 100K on an EIGER2 16M detector at beamline X10SA at the Swiss Light Source (SLS). Data were processed and scaled using XDS and the structures solved using molecular replacement with MOLREP and the computational models of Sam0.2 and Sam0.7 as search models. Structures were completed by cyclic refinement with REFMAC5 and modelling using Coot. Data collection and refinement statistics are summarized together with PDB accession codes in Table 2.

*Nuclear magnetic resonance (NMR) spectroscopy*

Spectra were recorded at 310 K on Broker AVIII-600 and AVIII-800 spectrometers. Backbone sequential assignments were made using standard triple-resonance experiments, including 3D-(H)CC(CA)NH-TOCSY and 3D-(H)CC(CO)NH-TOCSY spectra. Sequential assignment was achieved for the majority of the protein, however a larger stretch between V36 and A64, which corresponds to the outer rim of the binding site, could not be assigned due to multiple signals for some residues and weak or missing signals for others. This is consistent with this region undergoing slow conformational exchange processes (i.e. over millisecond timescales). To test VEGF binding, ~50 $\mu$M $^{15}$N, $^{13}$C-labelled Sam0.2 was titrated with unlabelled VEGF at a molar ratio of 1:2 and 1:3. $^{15}$N-HSQC spectra were recorded at each titration point and compared with that of the free protein.

*HUVEC survival assay*

HUVECs (Sigma, 200-05N) were cultured in Endothelial Cell Growth Medium (EGM) (Cell Applications, Inc.,

211-500) and used at passages 4 to 6. 300 μl of cell suspension were seeded in a 48-well plate (Nunc, 150687) at a density of 105 cells/ml. When the culture reached about 80 % confluency, cells were washed once with DPBS and medium was changed to Endothelial Cell Basal Medium (EBM) (Cell Applications, Inc., 210-500) or EBM supplemented with 30 ng/ml rhVEGF, or with 30 ng/ml rhVEGF and different concentrations of VEGF inhibitors varying from 1 μg/ml to 100 μg/ml. Before the experiment, inhibitors were preincubated with rhVEGF for 30 min at room temperature. After incubation for 48 hours at 37°C, 5% CO2, 60 μl of CellTiter-Blue Reagent (Promega, G8080) were added to the wells and the plate was incubated for an additional 1 h under the same conditions. Cell survival was monitored by measuring fluorescence (560/590 nm) using a Synergy HTX Microplate Reader (BioTek). The results were normalized to the average of fluorescence values from the wells with EBM only.

*U937/HEK293T cell proliferation endpoint analysis*

U937 cells (DSMZ, ACC 5) were cultured in RPMI 1640 medium (Gibco, 22400071) supplemented with 10% FBS (Gibco, 10082147). HEK293T cells (DSMZ, ACC 635) were cultured in DMEM medium (Gibco, 41966029) supplemented with 10% FBS (Gibco, 10082147). Cells were pelleted by centrifugation at 300 g for 5 min, washed once with DPBS (Gibco, 14190144) and once with non-supplemented medium. After the last washing step, cells were resuspended in RPMI 1640 (for U937) or DMEM (for HEK293T) medium supplemented with 1 % FBS. 100 μl of U937 cell suspension were seeded in a 96-well plate (Sarstedt, 83.3925.500) at a density of $2 \times 10^5$ cells/ml. For HEK293T, 100 μl of cell suspension were seeded in a 96-well plate (Corning, 3596) at a density of $5 \times 10^4$ cells/ml. Different concentrations of Sam0.7 or Sima3.2 varying from 1 μg/ml to 100 μg/ml were added to the wells in triplicates. DPBS was added to the wells serving as an untreated control. After incubation for 72 h at 37°C, 5% $CO_2$, 20 μl of CellTiter-Blue Reagent (Promega, G8080) were added to the wells and the plate was incubated for an additional 1 h under the same conditions to allow cells to convert resazurin to resorufin. Cell viability was monitored by measuring fluorescence (560/590 nm) using a Synergy HTX Microplate Reader (BioTek). The average of fluorescence values of the culture medium background was subtracted from all fluorescence values of the experimental wells. The data were presented as a percentage of untreated control fluorescence values.

*In vitro 3D microvasculature analysis*

Human episomal iPSC line from a healthy female donor, E1 (ThermoFisher Scientific, A18945) was cultured on Geltrex-coated (Gibco, A1413302) plates in Essential 8 ™ Medium (Gibco, A1517001). Endothelial cells (ECs) and pericytes (PCs) were differentiated following established protocols. ECs were labeled with the pJG-IRBP-mCherry viral vector to enable live cell imaging. Differentiated ECs were cultured on 0.2% gelatin-coated plates (Sigma-Aldrich, G2500) in Endothelial Cell Growth Medium (PromoCell, C-22020) supplemented with 30 ng/mL vascular endothelial growth factor A (PeproTech, 100-20) and 20 ng/ml fibroblast growth factor 2 (PeproTech, 100-18B); PCs were cultured in Essential 6 ™ Medium (Gibco, A1516501) supplemented with 10% FBS. Confluent ECs and PCs were detached with Accumax ™ (Sigma-Aldrich, A7098) pelleted by centrifugation at 300 g for 3 min and resuspended in a fibrin gel composed of 5 mg/mL fibrinogen (Sigma-Aldrich, F6755) and 4 U/mL thrombin (Sigma-Aldrich, T9549), with a final cell concentration $3 \times 10^6$ cell/mL of each cell type. 3D fibrin drops were formed using 5 μL of cell suspension in 96-well plates (Falcon, 351172) and cultured in Endothelial Cell Growth Medium supplemented with 20 ng/ml fibroblast growth factor 2. Two concentrations of Sam0.7 (50 and 100 μg/ml) were added to the wells, with 5 repeats for each condition; Sam_ctrl protein was added to the wells as control. The microvasculature was grown for 7 days and then imaged with an Axio Observer Z1/7 (Zeiss) microscope. Fiji/ImageJ software was utilized to analyze the microvasculature parameters. The statistical analysis was done using Graph Pad Prism 10 software. All procedures were in accordance with the Helsinki Convention and approved by the Ethical Committee of the Eber-hard Karls University Tübingen (no. 396/2021BO2).

*Xenotransplantation of U937-GFP or LNZ308-GFP cells in zebrafish embryos*

Zebrafish lines were maintained according to standard protocols and handled in accordance with European Union animal protection directive 2010/63/EU and approved by the local government (Tierschutzgesetz §11, Abs. 1, Nr. 1, husbandry permit 35/9185.46/Uni TÜ). The role of the designed inhibitors in cell survival and proliferation was evaluated by xenotransplantation of U937-GFP and LNZ308-GFP cell lines in zebrafish embryo at 1.5 dpf. U937-GFP cells were generated by lentiviral transduction with lentiviral construct expressing GFP, pRRL.PPT.SF..i2GFPpre, kindly provided by A. Schambach, Hannover Medical School. LNZ308-GFP cells were generated by lentiviral transduction with a third generation GFP-expressing lentivirus based on the lentiviral vector pLJM1-EGFP (Plasmid #19319, Addgene). 1 nl of U937-GFP cell suspension was injected at a density of $2 \times 10^5$ cells/pL (around 200 cells) into the perivitelline space, whereas LNZ308-GFP glioma cells (1 nl, around 200 cells) were orthopically implanted into the brain of anesthesized embryos at 29 hpf. The embryos were incubated at 35°C after transplantation. After 4 to 5 hours alive embryos with good engraftment were injected with same volume (4 nl) of either control (PBS or 3.0 mg/ml of inactive protein mvn_cntrl as a negative control, or 25 mg/ml of Bevacizumab as a positive control) or inhibitors (3.5 mg/ml of Sam0.7, and 2.4 mg/ml of Sima3.2). Injections were done either in the brain or bloodstream of zebrafish embryos transplanted with LNZ308-GFP cells or U937-GFP cells, respectively. The embryos were positioned and orientated laterally within cavities formed in 1 % agarose on a 96-well plate for imaging. To quantify the level of transplanted human cells, zebrafish embryos were imaged by a Nikon fluorescent stereomicroscope (SMZ18). All images taken under same condition were analyzed with Imaris software. The fluorescently labeled cells were quantified by surface measurement option with background subtraction. These values were calculated in each embryo of each group and plotted using Grafpad. The statistical analysis was performed using Prism 7 software.

*Data accessibility*

Coordinates and structure factors of Sam0.2 and Sam0.7 were deposited in the PDB under the accession codes 8BL5 and 8BL9, respectively.

**Claims**

1. A polypeptide configured to assemble into an inhibitor of vascular endothelial growth factor (VEGF), said inhibitor specifically binds to the receptor binding site of VEGF.

2. The polypeptide of claim 1, which is a non-immunoglobulin-derived polypeptide.

3. The polypeptide of claim 1 or 2, which comprises an amino acid chain having approx. 100 - approx. 200 amino acids, preferably approx. 120 - approx. 180 amino acids, further preferably the'Sam' polypeptides (SEQ ID NOS: 3, 5-11) having approx. 100 - approx. 150 amino acids, preferably of approx. 110 - approx. 130 amino acids, most preferably of approx. 128 amino acids; and further approx. the 'Sima' polypeptides (SEQ ID NOS: 4, 12-18) having approx. 150 - approx. 200 amino acids, preferably of approx. 170 - approx. 180 amino acids, most preferably of approx. 177 and/or approx. 179 amino acids.

4. The polypeptide of any of the preceding claims, which comprises the following amino acid sequence SEQ ID NO: 1:

$$\text{LPTA}X_1\text{EVQ}X_2\text{LMARYIEL}X_3\text{D}X_4\text{GDIEAIVQMYADDATVEAPFG}X_5\text{PPI}$$
$$\text{HGRERIAYFYRRMLG}X_6\text{GIARATLTGPVRASHNGTGAMPFRVE}X_7\text{V}X_8\text{NGQP}X_9\text{AMD}X_{10}\text{RVEMRFDEHGRIQTMQAYW}X_{11}X_{12}\text{VN}X_{13}\text{SVREP},$$

wherein

$X_1$ = Q or E
$X_2$ = G or K
$X_3$ = M or L
$X_4$ = V or K
$X_5$ = A or S
$X_6$ = G or E
$X_7$ = F or Y
$X_8$ = F or L
$X_9$ = Y or F or V
$X_{10}$ = V or I
$X_{11}$ = S or D
$X_{12}$ = W or E
$X_{13}$ = L or I or V or D.

5. The polypeptide of any of the preceding claims, which comprises the following amino acid sequence SEQ ID NO: 2:

X₁X₂STNISPKQGLDKAKYFSGKWYLTHVLIKDP**X₃X₄**V**X₅**QFC**X₆**SFTP
RESDGTVK**X₇**A**X₈**Y**X₉**YLAIKKTS**X₁₀**YAIGEGKLESSGLQYTAT**X₁₁**K**X₁**
₂VDKKKAVLKE**X₁₃**DER**X₁₄**SYT**X₁₅**TVLEADDSSALTH**X₁₆X₁₇X₁₈**REGS
KDYGDYY**X₁₉**VLTHQKDAEPSAKVKSAVTQAGLQLSQFVGTKDLGC
QYDDQFTSL

wherein

$X_1$ = D or missing
$X_2$ = C or missing
$X_3$ = V or K
$X_4$ = A or I
$X_5$ = T or S
$X_6$ = S or A
$X_7$ = E or I
$X_8$ = I orV
$X_9$ = V or L
$X_{10}$ = E or D
$X_{11}$ =For S
$X_{12}$ =TorV
$X_{13}$ = Wor L
$X_{14}$ = Y or H
$X_{15}$ = I orV
$X_{16}$ = V or I
$X_{17}$ = C orT
$X_{18}$ = T or V
$X_{19}$ = M or L.

6. The polypeptide of any of the preceding claims, which comprises any of the following amino acid sequences: SEQ ID NO: 3 (Sam0.7), SEQ ID NO: 4 (Sima3.2), SEQ ID NO: 5 (Sam0.1), SEQ ID NO: 6 (Sam0.2), SEQ ID NO: 7 (Sam0.3), SEQ ID NO: 8 (Sam0.4), SEQ ID NO: 9 (Sam0.5), SEQ ID NO: 10 (Sam0.6), SEQ ID NO: 11 (Sam0.8), SEQ ID NO: 12 (Sima1.1), SEQ ID NO: 13 (Sima2.1), SEQ ID NO: 14 (Sima3.1) SEQ ID NO: 15 (Sima4.1), SEQ ID NO: 16 (Sima1.2), SEQ ID NO: 17 (Sima2.2), SEQ ID NO: 18 (Sima4.2).

7. The polypeptide of any of claims 1-6 for use as a medicament, preferably in the treatment of a disease, further preferably selected from the group consisting of: cancer, cardiovascular disease, neurologic disease, and ophthalmic disease.

8. A protein comprising the polypeptide of any of claims 1-7.

9. The protein of claim 8, comprising one polypeptide having any of the following amino acid sequences: SEQ ID NO: 3 (Sam0.7), SEQ ID NO: 5 (Sam0.1), SEQ ID NO: 6 (Sam0.2), SEQ ID NO: 7 (Sam0.3), SEQ ID NO: 8 (Sam0.4), SEQ ID NO: 9 (Sam0.5), SEQ ID NO: 10 (Sam0.6), SEQ ID NO: 11 (Sam0.8),
and/or
comprising one, two or four polypeptide(s) having any of the following amino acid sequences: SEQ ID NO: 4 (Sima3.2), SEQ ID NO: 12 (Sima1.1), SEQ ID NO: 13 (Sima2.1), SEQ ID NO: 14 (Sima3.1) SEQ ID NO: 15 (Sima4.1), SEQ ID NO: 16 (Sima1.2), SEQ ID NO: 17 (Sima2.2), SEQ ID NO: 18 (Sima4.2).

10. The protein of claim 8 or 9 for use as a medicament, preferably in the treatment of a disease, further preferably selected from the group consisting of: cancer, cardiovascular disease, neurologic disease, and ophthalmic disease.

11. A nucleic acid molecule encoding the polypeptide of any of claims 1-7 or the protein of claim 8 or 9, optionally linked to a promoter sequence.

12. A recombinant host cell comprising the polypeptide according to any of claims 1-7, or the protein of claim 8 or 9, and/or the nucleic acid of claim 11.

13. A pharmaceutical composition cell comprising the polypeptide according to any of claims 1-7, or the protein of claim 8 or 9, the nucleic acid of claim 11, or the recombinant host cell of claim 12.

14. A kit comprising:

   (a) a container comprising the polypeptide according to any of claims 1-7, or the protein of claim 8 or 9, the nucleic acid of claim 11, the recombinant host cell of claim 12, or the pharmaceutical composition according to claim 13, in solution or in lyophilized formulation;
   (b) optionally, a second container containing a diluent or reconstituting solution for the lyophilized formulation;
   (c) optionally, instructions for (i) use of the solution or (ii) reconstitution and/or use of the lyophilized formulation.

15. A use of the polypeptide according to any of claims 1-7 for reconstituting a VEGF-binding and/or VEGF-inhibiting protein, preferably a monomeric, dimeric or tetrameric protein.

**Fig. 1**

**Fig. 2**

**A)**

forward map    inverse map

forward map

inverse map

**B)**

binder
forward map 1

target
inverse map 1

binder
forward map 2

target
inverse map 2

# Fig. 3

**A)**

R-factor = 0.526

VEGF inverse map          1PM1 forward map

**B)**

R-factor = 1.996

VEGF inverse map          1PM1 forward map

# Fig. 4

PDB: 1C7N

PDB: 1EYN

PDB: 1F73

PDB: 1KCD

PDB: 1OH0

PDB: 1PM1

# Fig. 5

Fig. 6

A)

B)

Fig. 7

Fig. 8

**Fig. 9**

Fig. 10

## A)

Sam0.7 (111, 134, 161, 193, 231 nM) binding to VEGF

$K_d$ = 189.6 $nM$
$K_a$ = 162955.1 $M^{-1}s^{-1}$
$K_d$ = 0.03089 $s^{-1}$
$R^2$ = 0.996 $x^2$ = 4.2

Sam0.7 binding to VEGF

y = 0.000163x+0.030892
$R^2$ = 0.978
p-value = 0.001

## B)

Sima3.2 (44, 66, 100, 148, 222 nM) binding to VEGF

$K_d$ = 13.7 $nM$
$K_a$ = 43714.3 $M^{-1}s^{-1}$
$K_d$ = 0.00060 $s^{-1}$
$R^2$ = 1.000 $x^2$ = 2.3

Sima3.2 binding to VEGF)

y = 0.000044x+0.000600
$R^2$ = 0.944
p-value = 0.006

## Fig. 11

A)

Sam0.7

B)

Fig. 12

A)

B)

Sam0.2

Fig. 13

**A)**

**B)**

**C)**

**D)**

Fig. 14

A)

Sam0.7 HUVEC survival (48h)

Sima3.2 HUVEC survival (48h)

B)

U937 proliferation (72h)

Fig. 15

## A)

Sam0.7 U937 proliferation (72h)

$IC_{50}$ = 8.0 µg/ml (533 nM)
$R^2$ = 0.989

## B)

Sima3.2. U937 proliferation (72h)

$IC_{50}$ = 3.6 µg/ml (180 nM)
$R^2$ = 0.983

# Fig. 16

**Fig. 17**

**A)**

**B)**

**C)**

# Fig. 18

**Fig. 19**

Fig. 20

Fig. 21

Fig. 22

Europäisches
Patentamt
European
Patent Office
Office européen
des brevets

## EUROPEAN SEARCH REPORT

**Application Number**

EP 24 17 3541

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/014830 A2 (COSMIX THERAPEUTICS LLC [US]; LOW DON [US] ET AL.) 4 February 2010 (2010-02-04) * peptide G2226 * * examples 5, 6 * * page 69, paragraph 3 * * sequence 28 * * page 66, line 1 - line 2 * | 1-3,7,8, 10-15 | INV. A61K38/00 A61K38/16 C07K14/00 C07K14/705 A61K38/18 |
| X | US 2009/035317 A1 (DAUGHERTY PATRICK S [US] ET AL) 5 February 2009 (2009-02-05) * paragraph [0150] * | 1,2,7,8, 10-15 | |
| X | FAIRBROTHER W J ET AL: "NOVEL PEPTIDES SELECTED TO BIND VASCULAR ENDOTHELIAL GROWTH FACTOR TARGET THE RECEPTOR-BINDING SITE", BIOCHEMISTRY, AMERICAN CHEMICAL SOCIETY, vol. 37, no. 51, 22 December 1998 (1998-12-22), pages 17754-17764, XP000876734, ISSN: 0006-2960, DOI: 10.1021/BI981931E * figures 2-4 * * table 1 * * abstract * | 1,2,7,8, 10-15 | **TECHNICAL FIELDS SEARCHED (IPC)**<br><br>A61K<br>C07K |
| X<br><br>A | US 6 348 333 B1 (NIWA MIKIO [JP] ET AL) 19 February 2002 (2002-02-19) * column 3, line 38 - line 43 * * column 4, line 1 - line 22 * * sequence 14 * | 1,7,8,10<br><br>11-15 | |

-/--

The present search report has been drawn up for all claims

| Place of search | Date of completion of the search | Examiner |
|---|---|---|
| The Hague | 18 September 2024 | Schmitz, Till |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt
European
Patent Office
Office européen
des brevets

# EUROPEAN SEARCH REPORT

**Application Number**

EP 24 17 3541

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PIOSSEK C ET AL: "VASCULAR ENDOTHELIAL GROWTH FACTOR VEGF) RECEPTOR II-DERIVED PEPTIDES INHIBIT VEGF", JOURNAL OF BIOLOGICAL CHEMISTRY, AMERICAN SOCIETY FOR BIOCHEMISTRY AND MOLECULAR BIOLOGY, US, vol. 274, no. 9, 26 February 1999 (1999-02-26), pages 5612-5619, XP002946875, ISSN: 0021-9258, DOI: 10.1074/JBC.274.9.5612 * figure 9 * * table 1 * * abstract * | 1,7,8, 10-15 | |
| X | MAKSYMENKO: "Award Winners and Abstracts of the 35 th Anniversary Symposium of The Protein Society, July 7-14, 2021 ¦ Virtual", PROTEIN SCIENCE, vol. 30, no. S1, 1 October 2021 (2021-10-01), pages 16-190, XP093206101, US ISSN: 0961-8368, DOI: 10.1002/pro.4191 * abstract 140; page 61 * | 1-3,7,8, 10-15 | **TECHNICAL FIELDS SEARCHED (IPC)** |
| A | WO 2019/101926 A1 (UNIV MANCHESTER [GB]) 31 May 2019 (2019-05-31) * sequence 2 * | 1-4,6-15 | |

-/--

The present search report has been drawn up for all claims

| Place of search | Date of completion of the search | Examiner |
|---|---|---|
| The Hague | 18 September 2024 | Schmitz, Till |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                
& : member of the same patent family, corresponding document

EPO FORM 1503 03.82 (P04C01)

Europäisches
Patentamt

European
Patent Office

Office européen
des brevets

**EUROPEAN SEARCH REPORT**

**Application Number**

EP 24 17 3541

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | CAO LONGXING ET AL: "Design of protein-binding proteins from the target structure alone", NATURE, vol. 605, no. 7910, 19 May 2022 (2022-05-19), pages 551-560, XP093149269, ISSN: 0028-0836, DOI: 10.1038/s41586-022-04654-9 Retrieved from the Internet: URL:https://www.nature.com/articles/s41586-022-04654-9.pdf> * the whole document * ----- | 1-4,6-15 | |
| A,D | MULLER Y A ET AL: "Vascular endothelial growth factor: crystal structure and functional mapping of the kinase domain receptor binding site", PROCEEDINGS OF THE NATIONAL ACADEMY OF SCIENCES, NATIONAL ACADEMY OF SCIENCES, vol. 94, no. 14, 8 July 1997 (1997-07-08), pages 7192-7197, XP002319823, ISSN: 0027-8424, DOI: 10.1073/PNAS.94.14.7192 * the whole document * ----- | 1-4,6-15 | |

TECHNICAL FIELDS SEARCHED (IPC)

The present search report has been drawn up for all claims

| Place of search | Date of completion of the search | Examiner |
|---|---|---|
| The Hague | 18 September 2024 | Schmitz, Till |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt

European
Patent Office

Office européen
des brevets

**Application Number**

EP 24 17 3541

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

4(completely); 1-3, 6-15(partially)

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION
SHEET B**

The Search Division considers that the present European patent application does not comply with the requirements of unity of invention and relates to several inventions or groups of inventions, namely:

```
1. claims: 4(completely); 1-3, 6-15(partially)

   A polypeptide configured to assemble into an inhibitor of
   vascular endothelial growth factor (VEGF), said inhibitor
   specifically binds to the receptor binding site of VEGF,
   wherein the peptide is defined by SEQ ID NO:1 ("Sam").
   Further protein, nucleic acid, host cell, pharmaceutical
   composition, kit and use based thereon.
                       ---


2. claims: 5(completely); 1-3, 6-15(partially)

   As invention 1, but wherein the peptide is defined by SEQ ID
   NO:2 ("Sima").
                       ---
```

## ANNEX TO THE EUROPEAN SEARCH REPORT
## ON EUROPEAN PATENT APPLICATION NO.

EP 24 17 3541

This annex lists the patent family members relating to the patent documents cited in the above-mentioned European search report.
The members are as contained in the European Patent Office EDP file on
The European Patent Office is in no way liable for these particulars which are merely given for the purpose of information.

18-09-2024

| Patent document cited in search report | | | Publication date | Patent family member(s) | | | Publication date |
|---|---|---|---|---|---|---|---|
| WO 2010014830 | A2 | | 04-02-2010 | EP | 2324048 | A2 | 25-05-2011 |
| | | | | US | 2010093624 | A1 | 15-04-2010 |
| | | | | WO | 2010014830 | A2 | 04-02-2010 |
| US 2009035317 | A1 | | 05-02-2009 | NONE | | | |
| US 6348333 | B1 | | 19-02-2002 | JP | 3837748 | B2 | 25-10-2006 |
| | | | | US | 6348333 | B1 | 19-02-2002 |
| | | | | WO | 9831794 | A1 | 23-07-1998 |
| WO 2019101926 | A1 | | 31-05-2019 | NONE | | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CAO, L et al.** De novo design of picomolar SARS-CoV-2 miniprotein inhibitors.. *Science*, 2020, vol. 370 (6515), 426-431
- **CAO, L. et al.** Design of protein-binding proteins from the target structure alone.. *Nature*, 2022, vol. 605 (7910), 551-560
- **GAINZA, P et al.** De novo design of site-specific protein interactions with learned surface fingerprints.. *Nature*, 2023, vol. 617, 176-184
- **MULLER, Y.A et al.** Vascular endothelial growth factor: crystal structure and functional mapping of the kinase domain receptor binding site.. *Proc Natl Acad Sci USA*, 1997, vol. 94 (14), 7192-7
- **BROZZO, M.S et al.** Thermodynamic and structural description of allosterically regulated VEGFR-2 dimerization. *Blood*, 2012, vol. 119 (7), 1781-8
- **SCHNEIDMAN-DUHOVNY, D. et al.** PatchDock and SymmDock: servers for rigid and symmetric docking.. *Nucleic Acids Res*, 2005, vol. 33, W363-7
- **FLEISHMAN, S.J et al.** RosettaScripts: a scripting language interface to the Rosetta macromolecular modeling suite. *PLoS One*, 2011, vol. 6 (6), e20161

- **KALÉ, L et al.** NAMD2: Greater Scalability for Parallel Molecular Dynamics.. *Journal of Computational Physics*, 1999, vol. 151 (1), 283-312
- **SHEFFLER, W.** ; **D. BAKER**. RosettaHoles: rapid assessment of protein core packing for structure prediction, refinement, design, and validation. *Protein Sci*, 2009, vol. 18 (1), 229-39
- **SKOKOWA, J. et al.** A topological refactoring design strategy yields highly stable granulopoietic proteins. *Nature Communications*, 2022, vol. 13 (1), 2948
- **EIGAMACY, M. et al.** An Interface-Driven Design Strategy Yields a Novel, Corrugated Protein Architecture. *ACS Synth Biol*, 2018, vol. 7 (9), 2226-2235
- **ELGAMACY, M** ; **M. COLES** ; **A. LUPAS**. Asymmetric protein design from conserved supersecondary structures. *J Struct Biol*, 2018, vol. 204 (3), 380-387